# EUROPEAN PATENT APPLICATION

(11) **EP 4 636 150 A1**
(43) Date of publication of application: **22.10.2025**
(21) Application number: 23902673.5
(22) Date of filing: 12.12.2023
(51) Int. Cl.: D06F 34/10, D06F 34/08, D06F 37/00

(54) **APPARATUS FOR SUPPLYING POWER TO INTERIOR OF DRUM WASHING MACHINE BODY, AND WASHING MACHINE**

(30) Priority: 14.12.2022 CN 202211607494; 08.12.2023 CN 202311681683
(71) Applicant: Shenzhen Yimu Technology Co., Ltd., Shenzhen, Guangdong 518110 (CN); Nanjing Yimu Intelligent Technology Co., Ltd., Nanjing, Jiangsu 210000 (CN)
(72) Inventor: LI, Hongyi, Shenzhen, Guangdong 518110 (CN)
(74) Representative: Valet Patent Services Limited
(86) International application number: PCT/CN2023/138017
(87) International publication number: WO 2024/125477

(57) **Abstract**

The present disclosure discloses an apparatus for supplying power to interior of a drum body of a drum washing machine and a washing machine. The apparatus includes a conductive ring, a tripod, a supporting frame, as well as a telescopic electromagnet and a pogopin circuit board both electrically connected with a main control board of the washing machine. The supporting frame is provided on an outer wall of an outer tub of the washing machine. The conductive ring and the telescopic electromagnet are mounted opposite to each other on the supporting frame. The pogopin circuit board is mounted at an output end of the telescopic electromagnet. The telescopic electromagnet extends or retracts to drive the pogopin circuit board to change a position, thus achieving the conduction or disconnection of conductive electric wires on the conductive ring, and achieving the on or off of a power supply to various functional assemblies in the inner drum of the washing machine. The present disclosure is ingenious in design, and adopting a conducting method by contact and detachment ensures stable and reliable operation, thus effectively preventing device wear and extending the service life.

## Description

### TECHNICAL FIELD

The present disclosure belongs to the technical field of washing machines, and in particular, relates to an apparatus for supplying power to interior of a drum body of a drum washing machine, and a washing machine.

### BACKGROUND

At present, some existing washing machines include sterilization and drying functions. The sterilization and drying apparatuses of such washing machines are mounted in an outer tub and controlled to be turned on/off through an electric control board of the outer tub. If a gripping apparatus, a sterilization and drying apparatus, a position sensor, a temperature or humidity sensor and the like are mounted in an inner drum of the washing machine, the existing washing machine cannot achieve the on/off of these apparatuses, so that the functions of these apparatuses in the inner drum cannot be achieved.

### SUMMARY

**Technical purposes:** Aiming at the above technical problem, the present disclosure provides an apparatus for supplying power to interior of a drum body of a drum washing machine, and a washing machine. The design is ingenious, and adopting a conducting method by contact and detachment ensures stable and reliable operation, thus effectively preventing device wear and extending the service life.

**Technical solutions:** To achieve the above technical purposes, the present disclosure adopts the following technical solutions:
A first purpose of the present disclosure is to provide an apparatus for supplying power to interior of a drum body of a drum washing machine. The drum washing machine includes an inner drum, an outer tub, and a main control board of the washing machine. The apparatus includes a telescopic electromagnet, a pogopin circuit board, a locking nut, a conductive ring, a fixing block, a tripod, and a supporting frame. The supporting frame is provided on an outer wall of the outer tub. An inner drum rib protruding to the interior of the inner drum is provided on an inner wall of the inner drum. A plurality of functional assemblies are provided in the inner drum. A bottom end of the tripod is fixed on an outer side of the inner drum, and a top end penetrates through the outer tub and is fixedly connected with the supporting frame.

The fixing block penetrates through the supporting frame and is connected with the top end of the tripod. An outer side of the fixing block is provided with the locking nut. The conductive ring and the telescopic electromagnet are mounted opposite to each other on the supporting frame. The pogopin circuit board is mounted at an output end of the telescopic electromagnet. The telescopic electromagnet and the pogopin circuit board are both electrically connected with the main control board of the washing machine. The telescopic electromagnet extends or retracts to drive the pogopin circuit board to change a position to connect or separate the pogopin circuit board and the conductive ring.

Preferably, the pogopin circuit board is provided with a plurality of pogopins. The conductive ring is provided with a plurality of metal conductive sheets corresponding to the pogopins and a plurality of conductive electric wires corresponding to the metal conductive sheets. Insulating plastic is provided between adjacent metal conductive sheets. One ends of the conductive electric wires are electrically connected with the metal conductive sheets, and the other ends penetrate through the supporting frame and a central passage in the tripod, enter an outer side of the inner drum rib and are respectively connected with power supply ends of the functional assemblies.

Preferably, the metal conductive sheets are of a ring-shaped structure. The conductive electric wires penetrate through central holes of the metal conductive sheets and enter the central passage of the tripod, then the central passage is sealed with liquid silica gel, and the silica gel is solidified to seal the central passage.

Preferably, the pogopin circuit board is provided with three pogopins, and the conductive ring is provided with three metal conductive sheets and three conductive electric wires.

Preferably, the apparatus further includes a DD motor configured to drive the inner drum to rotate. A motor rotor of the DD motor sleeves the tripod. The DD motor is mounted between the outer tub and the supporting frame, and a rotation speed of the DD motor is fed back to the main control board of the washing machine in real time.

The main control board of the washing machine controls the on/off of the power supply to the telescopic electromagnet and the pogopin circuit board according to a current washing process and a state of the DD motor.

Preferably, the telescopic electromagnet includes an electromagnet body, a cylindrical protrusion, a spring, and a telescopic rod. A center of the electromagnet body is provided with a through hole. One end of the telescopic rod penetrates through the through hole and is provided with the spring at an end portion, and the cylindrical protrusion is mounted at the other end. The cylindrical protrusion serves as the output end of the telescopic electromagnet.

Preferably, the functional assemblies include one or more of a gripping apparatus, a sterilization and drying apparatus, a position sensor, a temperature sensor, and a humidity sensor.

A second purpose of the present disclosure is to provide a washing machine, which includes an inner drum, an outer tub, and a main control board of the washing machine. The inner drum is provided with a plurality of functional assemblies, and the working power supply to the functional assemblies is provided by the apparatus for supplying power to the interior of the drum body of the drum washing machine.

A third purpose of the present disclosure is to provide an apparatus for supplying power to interior of a drum body of a drum washing machine, which includes:
an inner drum, configured to accommodate clothes;
an outer tub, mounted at an outer side of the inner drum;
a tripod, fixedly connected with a bottom of the inner drum to transmit a rotation driving force to the inner drum, where an end portion of the tripod extends along an axial direction of the inner drum and penetrates through the outer tub to form a rotating shaft;
a conductive ring, fixed to an end portion of the rotating shaft and electrically conducted with a functional assembly inside the drum body of the washing machine;
a bearing, where an inner ring is fitted and fixed to an outer wall of the conductive ring, and the bearing is configured to achieve synchronous rotation of the conductive ring with the inner ring of the bearing; and
at least one circuit board assembly, electrically connected with a main control board of the washing machine and mounted on an outer ring of the bearing, where electrical connecting ends are formed on the circuit board assembly and configured to be connected with the conductive ring.

**In** a process that the conductive ring rotates relative to the circuit board assembly, the electrical connecting ends are configured to be always in contact with the conductive ring to achieve power supply to the functional assembly inside the drum body of the washing machine.

Preferably, a surface of the conductive ring in contact with the electrical connecting ends is provided with a coating layer.

Preferably, a plurality of metal conductive areas with positions corresponding to the electrical connecting ends and a plurality of conducting wires corresponding to the metal conductive areas are formed on the conductive ring.

An insulating plate is provided between adjacent metal conductive areas.

One end of each conducting wire is electrically connected with the corresponding metal conductive area, and the other end penetrates through a wiring passage inside the tripod to enter the outer side of the inner drum and connect with a power supply end of the functional assembly.

Preferably, the tripod includes a tripod body and a rotating shaft, and the tripod body is fixedly connected with the bottom of the inner drum.

A first wiring space is formed in the rotating shaft, and a second wiring space is formed in the tripod body. The conducting wires sequentially penetrate through the first wiring space and the second wiring space, and exit from an end portion of the tripod body.

Preferably, the circuit board assembly includes a bracket and a circuit board mounted on the bracket.

The bracket is fixed to the outer ring of the bearing, and an opening is formed in a sidewall of the bracket.

The electrical connecting ends are formed on the circuit board, and the circuit board is mounted in the opening to make the electrical connecting ends be in contact with the conductive ring.

Preferably, the circuit board assembly includes at least three groups of electrical connecting ends for respectively achieving power supply, grounding, and signal line functions.

Preferably, the electrical connecting ends are elastic members, and end portions of the elastic members are maintained in contact with the conductive ring under an elastic action.

Preferably, two circuit board assemblies are provided and are clamped onto the bearing to form a ring-shaped structure to be fitted to a periphery of the conductive ring.

Preferably, two bearings are provided and are respectively provided at two ends of the conductive ring, and the circuit board assembly is clamped onto the two bearings.

Preferably, the apparatus for supplying power to the interior of the drum body of the drum washing machine further includes: an end cover, mounted at an end portion of the conductive ring and configured to fix the inner ring of the bearing from an outer side.

A fourth purpose of the present disclosure is to provide an internal drum body of a washing machine, which includes:
an inner drum, configured to accommodate clothes;
a tripod, fixedly connected with a bottom of the inner drum to transmit a rotation driving force to the inner drum;
a power actuating mechanism, disposed inside the inner drum to actuate a traction effect on the clothes; and
a power supply apparatus, located at one end of the tripod, where a wiring end of the power supply apparatus penetrates through a hollow portion inside the tripod and is connected with the power actuating mechanism.

The power supply apparatus is configured to maintain power supply to the power actuating mechanism in a process that the tripod drives the inner drum to rotate.

Preferably, the power actuating mechanism includes one or more of a gripping apparatus, a poking apparatus, a stirring apparatus, and a lifting apparatus.

Preferably, the power supply apparatus includes a conductive ring and a fixed wiring portion which are electrically connected.

The conductive ring is fixed at an end portion of the tripod and synchronously rotates along with the tripod.

The fixed wiring portion is mounted with a housing located outside the inner drum and is configured to contact the conductive ring to achieve electrical connection, so as to achieve electric conduction in a process that the conductive ring rotates relative to the fixed wiring portion.

Preferably, the fixed wiring portion includes a telescopic electromagnet and a pogopin circuit board.

The pogopin circuit board is mounted at an output end of the telescopic electromagnet.

The telescopic electromagnet extends and retracts to drive the pogopin circuit board to produce a positional change, so that the pogopin circuit board is connected with or separated from the conductive ring.

Preferably, the fixed wiring portion includes a bracket and a circuit board fixed to the bracket.

The bracket is rotatably mounted with the conductive ring through a bearing.

Electrical connecting ends are formed on one side of the circuit board towards the conductive ring, and are always in contact with a sidewall of the conductive ring to achieve electric conduction in a process that the conductive ring rotates relative to the bracket.

Preferably, the power actuating mechanism is mounted on a sidewall of the inner drum.

Preferably, the inner drum further includes a lifter, and the power actuating mechanism is mounted with the lifter.

Preferably, the power actuating mechanism is hidden in the lifter.

Preferably, the power actuating mechanism is mounted at the bottom of the inner drum.

A fifth purpose of the present disclosure is to provide an internal drum body of a washing machine, which includes:
an inner drum, configured to accommodate clothes;
a tripod, fixedly connected with a bottom of the inner drum to transmit a rotation driving force to the inner drum;
a sensing apparatus, disposed in the inner drum to sense environmental parameters in the inner drum; and
a power supply apparatus, located at one end of the tripod, where a wiring end of the power supply apparatus penetrates through a hollow portion inside the tripod and is connected with the sensing apparatus.

The power supply apparatus is configured to maintain power supply to the sensing apparatus in a process that the tripod drives the inner drum to rotate.

Preferably, the sensing apparatus includes one or more of a temperature sensor, a humidity sensor, a weight sensor, an acceleration sensor, a position sensor, a water level sensor, and a water quality sensor.

Preferably, the power supply apparatus includes a conductive ring and a fixed wiring portion which are electrically connected.

The conductive ring is fixed at an end portion of the tripod and synchronously rotates along with the tripod.

The fixed wiring portion is mounted with a housing located outside the inner drum and is configured to contact the conductive ring to achieve electrical connection, so as to achieve electric conduction in a process that the conductive ring rotates relative to the fixed wiring portion.

Preferably, the fixed wiring portion includes a telescopic electromagnet and a pogopin circuit board.

The pogopin circuit board is mounted at an output end of the telescopic electromagnet.

The telescopic electromagnet extends and retracts to drive the pogopin circuit board to produce a positional change, so that the pogopin circuit board is connected with or separated from the conductive ring.

Preferably, the fixed wiring portion includes a bracket and a circuit board fixed to the bracket.

The bracket is rotatably mounted with the conductive ring through a bearing.

Electrical connecting ends are formed on one side of the circuit board towards the conductive ring, and are always in contact with a sidewall of the conductive ring to achieve electric conduction in a process that the conductive ring rotates relative to the bracket.

Preferably, the sensing apparatus is mounted on a sidewall of the inner drum.

Preferably, the inner drum further includes a lifter, and the sensing apparatus is fixedly mounted with the lifter.

Preferably, the sensing apparatus includes at least one sensing area, and the sensing area is embedded into a surface of the lifter.

Preferably, the sensing apparatus is mounted at a bottom of the inner drum.

A sixth purpose of the present disclosure is to provide an internal drum body of a washing machine, which includes:
an inner drum, configured to accommodate clothes;
a tripod, fixedly connected with a bottom of the inner drum to transmit a rotation driving force to the inner drum;
a heating apparatus, disposed in the inner drum to provide a thermal environment of the drum body; and
a power supply apparatus, located at one end of the tripod, where a wiring end of the power supply apparatus penetrates through a hollow portion inside the tripod and is connected with the heating apparatus.

The power supply apparatus is configured to maintain power supply to the heating apparatus in a process that the tripod drives the inner drum to rotate.

Preferably, the power supply apparatus includes a conductive ring and a fixed wiring portion which are electrically connected.

The conductive ring is fixed at an end portion of the tripod and synchronously rotates along with the tripod.

The fixed wiring portion is mounted with a housing located outside the inner drum and is configured to contact the conductive ring to achieve electrical connection, so as to achieve electric conduction in a process that the conductive ring rotates relative to the fixed wiring portion.

Preferably, the fixed wiring portion includes a telescopic electromagnet and a pogopin circuit board.

The pogopin circuit board is mounted at an output end of the telescopic electromagnet.

The telescopic electromagnet extends and retracts to drive the pogopin circuit board to produce a positional change, so that the pogopin circuit board is connected with or separated from the conductive ring.

Preferably, the fixed wiring portion includes a bracket and a circuit board fixed to the bracket.

The bracket is rotatably mounted with the conductive ring through a bearing.

Electrical connecting ends are formed on one side of the circuit board towards the conductive ring, and are always in contact with a sidewall of the conductive ring to achieve electric conduction in a process that the conductive ring rotates relative to the bracket.

Preferably, the heating apparatus is mounted on a sidewall of the inner drum.

Preferably, the inner drum further includes a lifter, and the heating apparatus is mounted in the lifter.

Preferably, the heating apparatus includes a heating tube, and the heating tube extends along a length direction of the lifter.

Preferably, the heating apparatus is mounted at a bottom of the inner drum.

Preferably, the heating apparatus is arranged close to the tripod.

A seventh purpose of the present disclosure is to provide an internal drum body of a washing machine, which includes:
an inner drum, configured to accommodate clothes;
a tripod, fixedly connected with a bottom of the inner drum to transmit a rotation driving force to the inner drum;
a sterilization apparatus, disposed inside the inner drum to perform sterilization and purification on the drum body; and
a power supply apparatus, located at one end of the tripod, where a wiring end of the power supply apparatus penetrates through a hollow portion inside the tripod and is connected with the sterilization apparatus.

The power supply apparatus is configured to maintain power supply to the sterilization apparatus in a process that the tripod drives the inner drum to rotate.

Preferably, the power supply apparatus includes a conductive ring and a fixed wiring portion which are electrically connected.

The conductive ring is fixed at an end portion of the tripod and synchronously rotates along with the tripod.

The fixed wiring portion is mounted with a housing located outside the inner drum and is configured to contact the conductive ring to achieve electrical connection, so as to achieve electric conduction in a process that the conductive ring rotates relative to the fixed wiring portion.

Preferably, the fixed wiring portion includes a telescopic electromagnet and a pogopin circuit board.

The pogopin circuit board is mounted at an output end of the telescopic electromagnet.

The telescopic electromagnet extends and retracts to drive the pogopin circuit board to produce a positional change, so that the pogopin circuit board is connected with or separated from the conductive ring.

Preferably, the fixed wiring portion includes a bracket and a circuit board fixed to the bracket.

The bracket is rotatably mounted with the conductive ring through a bearing.

Electrical connecting ends are formed on one side of the circuit board towards the conductive ring, and are always in contact with a sidewall of the conductive ring to achieve electric conduction in a process that the conductive ring rotates relative to the bracket.

Preferably, the sterilization apparatus is a light sterilization apparatus.

Preferably, the light sterilization apparatus is mounted on an inner sidewall of the inner drum to sterilize the clothes and washing water in the inner drum.

Preferably, the light sterilization apparatus is mounted on an outer sidewall of the inner drum to sterilize a space sandwiched between the inner drum and the outer tub.

Preferably, the inner drum further includes a lifter, and the light sterilization apparatus is mounted in the lifter.

Preferably, the light sterilization apparatus is mounted at a bottom of the inner drum.

Preferably, the light sterilization apparatus includes a UV sterilization apparatus or a light plasma sterilization apparatus.

Preferably, the sterilization apparatus is a hypochlorous acid sterilization apparatus.

Preferably, the hypochlorous acid sterilization apparatus is mounted on a sidewall of the inner drum.

Preferably, the inner drum further includes a lifter, and the hypochlorous acid sterilization apparatus is mounted in the lifter.

Preferably, the hypochlorous acid sterilization apparatus includes an electrolysis circuit board, a positive electrode end, and a negative electrode end.

The electrolysis circuit board is clamped on an inner side of the lifter and is connected with a wiring end of the power supply apparatus. The positive electrode end and the negative electrode end extend from a surface of the lifter and are respectively connected with a positive electrode and a negative electrode of the circuit board.

Preferably, the surface of the lifter is provided with two through holes for respectively clamping the positive electrode end and the negative electrode end.

Preferably, the hypochlorous acid sterilization apparatus is mounted at a bottom of the inner drum.

Preferably, the sterilization apparatus is a high-temperature steam sterilization apparatus.

Preferably, the high-temperature steam sterilization apparatus is mounted on a sidewall of the inner drum.

Preferably, the inner drum further includes a lifter, and the high-temperature steam sterilization apparatus is mounted in the lifter.

Preferably, the high-temperature steam sterilization apparatus includes a heating plate, and the heating plate extends along a length direction of the lifter.

Preferably, the high-temperature steam sterilization apparatus is mounted at a bottom of the inner drum.

An eighth purpose of the present disclosure is to provide an internal drum body of a washing machine, which includes:
an inner drum, configured to accommodate clothes;
a tripod, fixedly connected with a bottom of the inner drum to transmit a rotation driving force to the inner drum;
a steam generation apparatus, disposed in the inner drum to provide steam to the drum body; and
a power supply apparatus, located at one end of the tripod, where a wiring end of the power supply apparatus penetrates through a hollow portion inside the tripod and is connected with the steam generation apparatus.

The power supply apparatus is configured to maintain power supply to the steam generation apparatus in a process that the tripod drives the inner drum to rotate.

Preferably, the steam generation apparatus is a thick film steam generator.

Preferably, the power supply apparatus includes a conductive ring and a fixed wiring portion which are electrically connected.

The conductive ring is fixed at an end portion of the tripod and synchronously rotates along with the tripod.

The fixed wiring portion is mounted with a housing located outside the inner drum and is configured to contact the conductive ring to achieve electrical connection, so as to achieve electric conduction in a process that the conductive ring rotates relative to the fixed wiring portion.

Preferably, the fixed wiring portion includes a telescopic electromagnet and a pogopin circuit board.

The pogopin circuit board is mounted at an output end of the telescopic electromagnet.

The telescopic electromagnet extends and retracts to drive the pogopin circuit board to produce a positional change, so that the pogopin circuit board is connected with or separated from the conductive ring.

Preferably, the fixed wiring portion includes a bracket and a circuit board fixed to the bracket.

The bracket is rotatably mounted with the conductive ring through a bearing.

Electrical connecting ends are formed on one side of the circuit board towards the conductive ring, and are always in contact with a sidewall of the conductive ring to achieve electric conduction in a process that the conductive ring rotates relative to the bracket.

Preferably, the steam generation apparatus is mounted on a sidewall of the inner drum.

Preferably, the inner drum further includes a lifter, and the steam generation apparatus is mounted in the lifter.

Preferably, the steam generation apparatus includes a heating plate, and the heating plate extends along a length direction of the lifter.

Preferably, the steam generation apparatus is mounted at a bottom of the inner drum.

A ninth purpose of the present disclosure is to provide a washing machine, which includes the apparatus for supplying power to the interior of the drum body of the drum washing machine, or the internal drum body of the washing machine described above.

**Beneficial effects:** By adopting the above technical solutions, the present disclosure has the following beneficial effects:
The present disclosure adopts a conducting method by contact and detachment to achieve connection at low speeds and disconnection at high speeds; wear is prevented, and the service life is extended; the structure is simple, the operation is stable, and the reliability is high.

### BRIEF DESCRIPTION OF THE DRAWINGS

FIG. 1 is a schematic cross-sectional structural diagram of an apparatus for supplying power to interior of a drum body of a drum washing machine in Embodiment 1;
FIG. 2 is a schematic cross-sectional diagram that a pogopin circuit board is connected with a conductive ring in FIG. 1;
FIG. 3 is a right view of FIG. 1;
FIG. 4 is a three-dimensional diagram of FIG. 1;
FIG. 5 is a schematic diagram that a pogopin circuit board is connected with or disconnected from a conductive ring in an apparatus in FIG. 1;
FIG. 6 is a three-dimensional diagram of a conductive ring;
FIG. 7 is a front view of FIG. 6;
FIG. 8 is a schematic diagram of extended and compressed states of a telescopic electromagnet;
FIG. 9 is an overall schematic structural diagram of an apparatus for supplying power to interior of a drum body of a drum washing machine in Embodiment 3;
FIG. 10 is a sectional view of an apparatus for supplying power to interior of a drum body of a drum washing machine in Embodiment 3;
FIG. 11 is a schematic diagram I of an assembling relationship among a tripod, a conductive ring, a bearing, and a circuit board assembly in Embodiment 3;
FIG. 12 is a schematic diagram II of an assembling relationship among a tripod, a conductive ring, a bearing, and a circuit board assembly in Embodiment 3;
FIG. 13 is a sectional view of an assembled structure of a tripod, a conductive ring, a bearing, and a circuit board assembly in Embodiment 3;
FIG. 14 is a schematic diagram I of an assembling relationship among a conductive ring, a bearing, and a circuit board assembly in Embodiment 3;
FIG. 15 is a schematic structural diagram of a conductive ring in Embodiment 3;
FIG. 16 is a schematic diagram I of an assembling relationship between a power supply apparatus and an inner drum in Embodiment 4 to Embodiment 9;
FIG. 17 is a schematic diagram II of an assembling relationship between a power supply apparatus and an inner drum in Embodiment 4 to Embodiment 9;
FIG. 18 is a schematic diagram of an assembled structure of a conductive ring and conducting wires in Embodiment 4 to Embodiment 9;
FIG. 19 is a sectional view of an assembled structure of an inner drum, a lifter, and a power actuating mechanism in Embodiment 4;
FIG. 20 is a schematic diagram of an assembled structure of an inner drum, a lifter, and a power actuating mechanism in Embodiment 4;
FIG. 21 is a sectional view of an assembled structure of an inner drum, a lifter, and a sensing apparatus in Embodiment 5;
FIG. 22 is a schematic diagram I of an assembled structure of a sensing apparatus and a lifter in Embodiment 5;
FIG. 23 is a schematic diagram II of an assembled structure of a sensing apparatus and a lifter in Embodiment 5;
FIG. 24 is a sectional view of an assembled structure of an inner drum, a lifter, and a heating apparatus in Embodiment 6;
FIG. 25 is a schematic diagram of an assembled structure of a heating apparatus and a lifter in Embodiment 6;
FIG. 26 is a sectional view of an assembled structure of an inner drum, a lifter, and a UV sterilization apparatus in Embodiment 7;
FIG. 27 is a schematic diagram of an assembled structure of an inner drum, a lifter, and a UV sterilization apparatus in Embodiment 7;
FIG. 28 is a schematic diagram of an assembled structure of a UV sterilization apparatus and a lifter in Embodiment 7;
FIG. 29 is a sectional view of an assembled structure of an inner drum, a lifter, and a hypochlorous acid sterilization apparatus in Embodiment 8;
FIG. 30 is a schematic diagram of an assembled structure of an inner drum, a lifter, and a hypochlorous acid sterilization apparatus in Embodiment 8;
FIG. 31 is a schematic diagram of an assembled structure of a hypochlorous acid sterilization apparatus and a lifter in Embodiment 8;
FIG. 32 is a sectional view of an assembled structure of an inner drum, a lifter, and a steam generation apparatus in Embodiment 9; and
FIG. 33 is a schematic diagram of an assembled structure of a steam generation apparatus and a lifter in Embodiment 9.

### Description of reference signs:

1-telescopic electromagnet; 101-electromagnet body; 102-cylindrical protrusion; 103-spring; 104-telescopic rod;
2-pogopin circuit board; 3-locking nut; 4-conductive ring; 5-fixing block; 6-tripod; 7-rotor of DD motor; 8-supporting frame; 9-DD motor; 10-insulating plastic; 11-conductive electric wire; 12-metal conductive sheet; 13-outer tub; 14-inner drum;
300-washing machine; 310-inner drum; 320-outer tub;
330-flange plate group; 331-rotating shaft; 3311-first wiring space; 332-tripod; 3321-tripod body; 3322-cover plate; 3323-second wiring space; 3324-through hole; 340-conductive ring; 341-metal conductive area; 342-insulating plate; 343-conducting wire; 350-bearing; 360-circuit board assembly; 361-first circuit board assembly; 3611-first bracket; 3612-first circuit board; 362-second circuit board assembly; 3621-second bracket; 3622-second circuit board; 363-electrical connecting end; 364-opening; 370-end cover;
410-drum body; 411-inner drum; 4111-lifter; 412-outer tub;
420-tripod; 421-wiring passage; 422-rotating shaft;
430-power actuating mechanism;
440-power supply apparatus; 441-conductive ring; 4411-metal conductive area; 4412-conducting wire; 4413-insulating plate; 442-fixed wiring portion; 4421-telescopic electromagnet; 44211-electromagnet body; 44212-telescopic rod; 4422-pogopin circuit board; 4423-bracket; 44231-opening; 4424-circuit board; 44241-electrical connecting end; 443-bearing;
510-drum body; 511-inner drum; 5111-lifter; 51111-through hole; 512-outer tub;
520-tripod; 521-wiring passage; 522-rotating shaft;
530-sensing apparatus; 531-sensing area; 532-sensing circuit board;
540-power supply apparatus; 541-conductive ring; 5411-metal conductive area; 5412-conducting wire; 5413-insulating plate; 542-fixed wiring portion; 5421-telescopic electromagnet; 54211-electromagnet body; 54212-telescopic rod; 5422-pogopin circuit board; 5423-bracket; 54231-opening; 5424-circuit board; 54241-electrical connecting end; 543-bearing;
610-drum body; 611-inner drum; 6111-lifter; 61111-water hole; 612-outer tub;
620-tripod; 621-wiring passage; 622-rotating shaft;
630-heating apparatus; 631-heating tube; 632-connecting portion;
640-power supply apparatus; 641-conductive ring; 6411-metal conductive area; 6412-conducting wire; 6413-insulating plate; 642-fixed wiring portion; 6421-telescopic electromagnet; 64211-electromagnet body; 64212-telescopic rod; 6422-pogopin circuit board; 6423-bracket; 64231-opening; 6424-circuit board; 64241-electrical connecting end; 643-bearing;
710-drum body; 711-inner drum; 7111-lifter; 71111-through hole; 712-outer tub;
720-tripod; 721-wiring passage; 722-rotating shaft;
730-UV sterilization apparatus; 731-UV lamp holder; 732-UV circuit board;
740-power supply apparatus; 741-conductive ring; 7411-metal conductive area; 7412-conducting wire; 7413-insulating plate; 742-fixed wiring portion; 7421-telescopic electromagnet; 74211-electromagnet body; 74212-telescopic rod; 7422-pogopin circuit board; 7423-bracket; 74231-opening; 7424-circuit board; 74241-electrical connecting end; 743-bearing;
810-drum body; 811-inner drum; 8111-lifter; 81111-through hole; 812-outer tub;
820-tripod; 821-wiring passage; 822-rotating shaft;
830-hypochlorous acid sterilization apparatus; 831-electrolysis circuit board; 832-positive electrode end; 833-negative electrode end;
840-power supply apparatus;
841-conductive ring; 8411-metal conductive area; 8412-conducting wire; 8413-insulating plate; 842-fixed wiring portion; 8421-telescopic electromagnet; 84211-electromagnet body; 84212-telescopic rod; 8422-pogopin circuit board; 8423-bracket;
84231-opening; 8424-circuit board; 84241-electrical connecting end; 843-bearing;
910-drum body; 911-inner drum; 9111-lifter; 91111-through hole; 912-outer tub;
920-tripod; 921-wiring passage; 922-rotating shaft;
930-steam generation apparatus; 931-thick film heating plate; 932-heating circuit board;
940-power supply apparatus; 941-conductive ring; 9411-metal conductive area; 9412-conducting wire; 9413-insulating plate; 942-fixed wiring portion; 9421-telescopic electromagnet; 94211-electromagnet body; 94212-telescopic rod; 9422-pogopin circuit board; 9423-bracket; 94231-opening; 9424-circuit board; 94241-electrical connecting end; and 943-bearing.

### DETAILED DESCRIPTION

The following describes embodiments of the present disclosure in detail with reference to the accompanying drawings.

### Embodiment 1

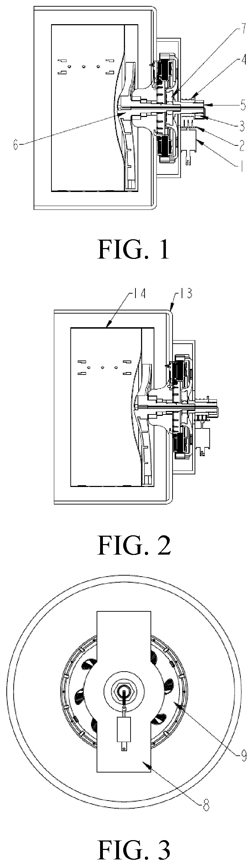

The present disclosure provides an apparatus for supplying power to interior of a drum body of a drum washing machine. As shown in FIG. 1 to FIG. 7, the apparatus includes: a pogopin circuit board 2, a locking nut 3, a conductive ring 4, a fixing block 5, a tripod 6, a supporting frame 8, a DD motor 9, and the like.

A telescopic electromagnet is mainly composed of an electromagnet 101, a telescopic rod 104, a spring 103, and a cylindrical protrusion 102. The cylindrical protrusion 102 is fixed at a front end of the telescopic rod, and the spring is inserted into a rear end of the telescopic rod. The diameter of a cylinder at the rear end is larger than that of a through hole in the electromagnet, so that it can be clamped to ensure that the telescopic rod can maintain a set stroke distance when popping out. The through hole is formed in a center of the electromagnet. The spring is fitted onto the telescopic rod and then inserted into the through hole of the electromagnet, and then the cylindrical protrusion 102 is fixed.

The working flow of the telescopic electromagnet 1 is as follows:
Power off: when it is not powered on, the telescopic electromagnet is in a non-working state, and the telescopic rod is in a non-popping-out state and is retracted in the electromagnet.
Power on: when a main control board is powered on, the telescopic electromagnet is in a working state, the telescopic rod is subjected to the magnetic force of the electromagnet, the magnetic force is greater than the elastic force of the spring, the telescopic rod pops out and maintains the set stroke distance.

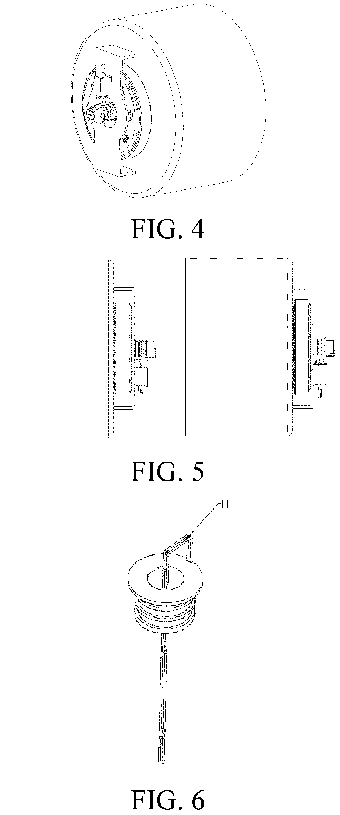

Specifically, the cylindrical protrusion 102 is fixedly connected with the pogopin circuit board 2, the pogopin circuit board 2 moves synchronously with the telescopic rod on the telescopic electromagnet, and the telescopic electromagnet 1 and the pogopin circuit board 2 are both electrically connected with the main control board of the washing machine. The telescopic electromagnet 1 is fixed on the supporting frame 8. The main control board is powered on, so the telescopic electromagnet is powered on and moves. Several pogopins arranged on the pogopin circuit board 2 move accordingly to contact the conductive ring. This embodiment is described by taking three pogopins as an example. The telescopic electromagnet is powered off and does not move, and the three pogopins on the pogopin circuit board 2 do not contact the conductive ring, as shown in FIG. 5.

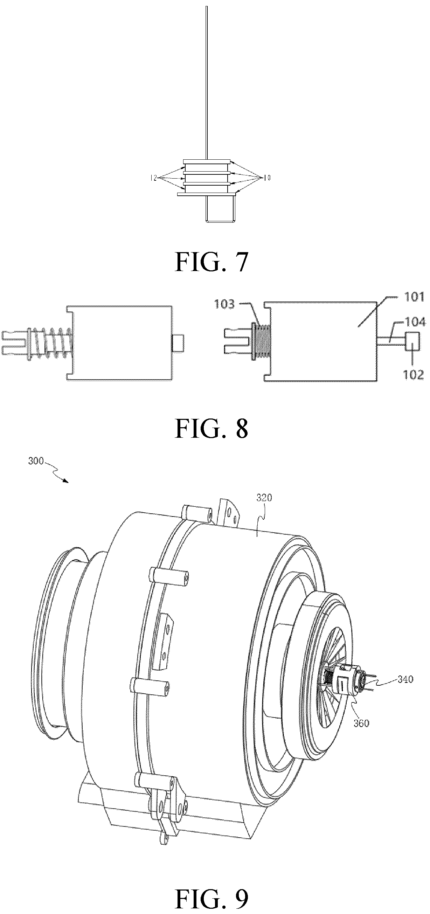

The conductive ring 4 is mainly composed of insulating plastics 10, a conductive electric wire 11, and a metal conductive sheet 12. As shown in FIG. 6 and FIG. 7, the metal conductive sheet 12 is composed of three ring-shaped metal conductive sheets; the conductive electric wire 11 is composed of three conducting wires; the three conducting wires of the conductive electric wire 11 are respectively connected with the three ring-shaped metal conductive sheets of the metal conductive sheet 12; the three ring-shaped metal conductive sheets of the metal conductive sheet 12 are embedded into an insulating body; and the insulating body contains four insulating plastics 10 to space apart the three ring-shaped metal sheets of the metal conductive sheet 12. The conductive ring 4 has a through hole in the middle, and the conductive electric wire 11 bends from one side and penetrates through the through hole to the inner drum.

When the apparatus for supplying power to the interior of the drum body of the drum washing machine in this embodiment is mounted, a DD motor rotor 7 is fitted onto the tripod 6, internal threads on the fixing block 5 (the fixing block contains the internal threads) are connected with external threads on a head of the tripod 6 and are tightened, and the DD motor 9 is tightly pressed. The DD motor is located on an outer side of the outer tub and is connected to the inner drum through a separate wire. The DD motor is configured to drive the inner drum to rotate. The conductive ring 4 is fitted onto the fixing block 5. The locking nut 3 matches with and is tightened to external threads on an end portion of the fixing block 5. The spacing between the three pogopins on the pogopin circuit board 2 is the same as the spacing between the three independent ring-shaped metal conductive sheets of the metal conductive sheet 12, and each pogopin is located in the middle of the corresponding ring-shaped metal conductive sheet. After the conductive electric wire 11 penetrates through the central through hole of the tripod, it is sealed with liquid silica gel. After the silica gel is cured, a central hole region of the tripod is completely sealed.

The working principle of the apparatus for supplying power to the interior of the drum body of the drum washing machine in this embodiment is as follows:
When the washing machine is in washing and rinsing/spinning programs, there is no need to supply power to devices (i.e., functional assemblies, such as a gripping apparatus, a sterilization and drying apparatus, a position sensor, a temperature or humidity sensor) in the inner drum. The main control board does not supply power to the telescopic electromagnet 1, the telescopic electromagnet 1 does not work, and the telescopic rod is in a non-popping-out state. Since the telescopic electromagnet 1 is fixedly connected with the cylindrical protrusion 102 at the front end of the telescopic rod, the pogopin circuit board 2 and the telescopic rod both do not pop out and are far away from the conductive ring 4, and the pogopins are not in contact with the conductive ring 4. The conductive circuit is in a disconnected state, and the devices in the inner drum are in a non-powered state.

When the washing machine is in drying and sterilization programs, power needs to be supplied to the devices inside the inner drum. The main control board supplies power to the telescopic electromagnet 1, and the telescopic electromagnet 1 starts working. The telescopic rod 104 pops out to a set stroke, and the pogopins pop out to designated positions accordingly. (Since the spring is built in the head of each pogopin, pogopin has a compressive conductivity feature, and with wear over time, unworn parts may pop out under the elastic force, maintaining good conductivity.) A contact end of the head of each pogopin is in contact and conduction with the ring-shaped metal conductive sheet on the conductive ring 4. Since the ring-shaped metal conductive sheet is independently connected with the conductive electric wire 11, the external is conducted with the internal circuit of the inner drum through the conductive electric wire 11 to achieve the purpose of power supply. The devices in the inner drum are powered on to execute the drying and sterilization programs.

In the present disclosure, the use of the pogopin circuit board has the following advantages: pogopin connectors are small in size, more space can be saved, pogopin contacts are more precise, the forward force is smaller than that of ordinary metal spring sheets when the compression stroke changes, and the reliability is higher. In addition, the pogopin product has a long service life of at least 10000 times, usually up to 100000 times. The spring is built in the pogopin, the spring will push the worn end to a position before the wear when wear occurs on the head, the impedance stability of the product can usually be controlled within 30 milliohms, it can also be controlled within 50 milliohms after service life testing, and it is an ideal connector that is resistant to high current, corrosion and the like. Therefore, when applied to the power supply apparatus inside the drum, the service life of the washing machine may be better extended, and the overall performance of the washing machine is improved.

### Embodiment 2

This embodiment provides a washing machine, which includes an inner drum, an outer tub, and a washing machine main control board. The inner drum is internally provided with a plurality of functional assemblies. A working power supply to the functional assemblies is provided by the apparatus for supplying power to the interior of the drum body of the drum washing machine.

Preferably, a driving motor for driving the inner drum to rotate is a DD motor. The DD motor is located at an outer side of the outer tub and is connected to the inner drum through a separate wire, and the DD motor is configured to drive the inner drum to rotate.

The present disclosure adopts a contact and disengagement conducting method to achieve power supply to various functional assemblies in the inner drum. It is turned on when the inner drum rotates at low speed and turned off when the inner drum rotates at high speed, that is, a current rotating speed is fed back to the main control board in real time through the DD motor. When the program of the washing machine is in any stage before the drying program (washing, spinning, or the like), the rotating speed of the inner drum is generally high because the inner drum is driven by the DD motor, and the rotating speed of the DD motor is also relatively high. During the above stages, the main control board may continue to disconnect the power supply to the telescopic electromagnet, pogopins cannot be in contact with the conductive ring for conduction, and are not in contact when not in use, preventing the conductive ring from being worn. When the program of the washing machine reaches the drying program, the DD motor may reduce the rotating speed. When the electrical devices in the drum need to work, the main control board may turn on the power supply to the telescopic electromagnet, and pogopins is in contact with the conductive ring for conduction.

### Embodiment 3

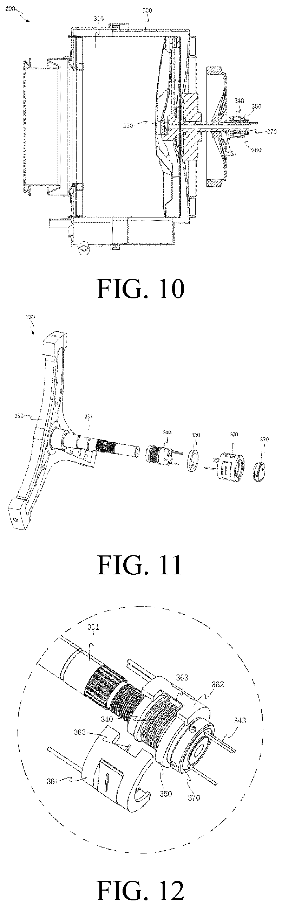

An embodiment of the present disclosure provides an apparatus for supplying power to interior of a drum body of a drum washing machine. As shown in FIG. 9 to FIG. 15, the apparatus includes:
an inner drum 310, configured to accommodate clothes;
an outer tub 320, mounted at an outer side of the inner drum 310;
a tripod 330, fixedly connected with a bottom of the inner drum 310 to transmit a rotation driving force to the inner drum 310, where an end portion of the tripod 330 extends along an axial direction of the inner drum 310 and penetrates through the outer tub 320 to form a rotating shaft 331;
a conductive ring 340, fixed to an end portion of the rotating shaft 331 and electrically conducted with a functional assembly inside a drum body of a washing machine 300;
a bearing 350, where an inner ring is fitted and fixed to an outer wall of the conductive ring 340, and the bearing is configured to achieve synchronous rotation of the conductive ring 340 with the inner ring of the bearing 350; and
at least one circuit board assembly 360, electrically connected with a main control board of the washing machine 300 and mounted on an outer ring of the bearing 350, where electrical connecting ends 363 are formed on the circuit board assembly 360 and configured to be connected with the conductive ring 340.

**In** a process that the conductive ring 340 rotate relative to the circuit board assembly 360, the electrical connecting ends 363 are configured to be always in contact with the conductive ring 340 to achieve power supply to the functional assembly inside the drum body of the washing machine 300.

Specifically, when the washing machine 300 works, the tripod 330 drives the inner drum 310 to rotate under the driving of a driving motor; the conductive ring 340 fixed at the end portion of the rotating shaft 331 rotates together with the tripod 330 and the inner drum 310; the circuit board assembly 360 fixed on the outer ring of the bearing 350 of the rotating shaft 331 remains stationary relative to the outer tub 320; and during the rotation of the conductive ring 340, the electrical connecting ends 363 are in sliding contact with the conductive ring 340 to maintain a conducted state.

This embodiment achieves power supply to the functional assembly inside the inner drum 310 when the inner drum 310 rotates by arranging the conductive ring 340 and the circuit board assembly 360. **In** this way, the cooperation between the rotating inner drum 310 and the functional assembly can expand functions of the washing machine 300, optimize overall functions of the washing machine 300, meet different needs of users, and improve user experience. **In** addition, such arrangement can effectively avoid entanglement of conducting wires during rotation, the structure is simple, the operation is stable, and the reliability is high.

**In** an embodiment, a surface of the conductive ring 340 in contact with the electrical connecting end 363 is provided with a coating layer.

Specifically, the conductive ring 340 is made of a metal material. The conductive ring 340 is electroplated. Inert material plating such as gold plating, titanium plating and chromium plating may be adopted to give the surface of the conductive ring 340 hardness and thickness that meet the service life requirements, so as to achieve wear resistance, high temperature resistance and aging resistance, thus extending the service life of the conductive ring 340.

In this embodiment, the surface of the conductive ring 340 is provided with a hardened chromium plating layer.

Specifically, chromium plating can increase the hardness and wear resistance of the metal surface, and improve the durability and wear resistance of metal parts. Therefore, using chromium plating can increase the wear resistance of the conductive ring 340 while reducing the frictional force of the electrical connecting ends 363 sliding on the conductive ring 340. Further, metal chromium plating can improve the conductivity of the metal surface. Therefore, this embodiment can improve the wear resistance of the conductive ring 340 and optimize the overall performance of the conductive ring 340 while ensuring the conductivity of the conductive ring 340.

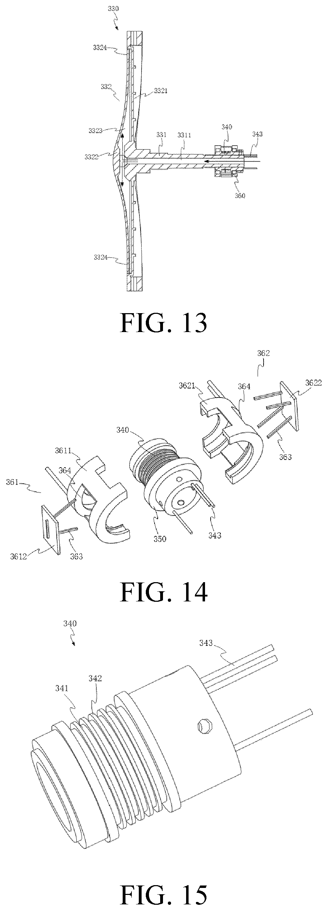

In an embodiment, as shown in FIG. 13, FIG. 14 and FIG. 15, a plurality of metal conductive areas 341 with positions corresponding to the electrical connecting ends 363 and a plurality of conducting wires 343 corresponding to the metal conductive areas 341 are formed on the conductive ring 340.

An insulating plate 342 is provided between adjacent metal conductive areas 341.

One end of each conducting wire 343 is electrically connected with the corresponding metal conductive area 341, and the other end penetrates through a wiring passage inside the tripod 330 to enter the outer side of the inner drum 310 and connect with a power supply end of the functional assembly.

In this embodiment, the conductive rings 340 and the electrical connecting ends 363 are in one-to-one correspondence. The metal conductive areas 341 are ring-shaped. The insulating plate 342 is also ring-shaped and is embedded into the surface of the conductive ring 340 to space apart adjacent metal conductive areas 341.

In an embodiment, as shown in FIG. 13, a hollow portion is formed in the tripod 330 to form the wiring passage.

Further, the tripod 330 includes a tripod body 332 and a rotating shaft 331, and the tripod body 332 is fixedly connected with the bottom of the inner drum 310.

A first wiring space 3311 is formed in the rotating shaft 331, and a second wiring space 3323 is formed in the tripod body 332. The conducting wires 343 sequentially penetrate through the first wiring space 3311 and the second wiring space 3323, and exit from an end portion of the tripod body 332. The arrows in FIG. 13 represent threading paths.

Specifically, the first wiring space 3311 is of a central passage structure formed by penetrating along an axial direction of the rotating shaft 331, and the second wiring space 3323 extends radially and matches the shape of the tripod body 332, that is, after entering the second wiring space 3323 from the first wiring space 3311, the conducting wires 343 can exit from any one or more of three directions enclosed by the tripod body 332, so as to adapt to the layout of the functional assembly in the inner drum 310.

Further, end portions of the tripod body 332 are provided with through holes 3324 to facilitate the conducting wires 343 to pass, which is beneficial for the tidiness of the wiring hamess.

Further, the tripod body 332 includes a supporting frame 3321 and a cover plate 3322 that are clamped to each other, and the supporting frame 3321 and the cover plate 3322 enclose to form the second wiring space 3323. The cover plate 3322 is located at a bottom of the tripod body 332 near the bottom of the inner drum 310. The cover plate 3322 that can be disassembled and assembled facilitates the layout of the conducting wires 343 and simplifies the assembling process.

Preferably, after the conducting wires 343 penetrate through the wiring passage, the wiring passage is sealed with liquid silica gel, and the silica gel is cured to seal the wiring passage.

In an embodiment, as shown in FIG. 13 and FIG. 14, the circuit board assembly 360 includes a bracket and a circuit board mounted on the bracket.

The bracket is fixed to the outer ring of the bearing 350, and an opening 364 is formed in a sidewall of the bracket.

The electrical connecting ends 363 are formed on the circuit board, and the circuit board is mounted in the opening 364 to make the electrical connecting ends 363 be in contact with the conductive ring 340.

Further, the shape of the opening 364 matches the shape of the circuit board, making it easier for the circuit board to be clamped into the opening 364 and achieving rapid disassembling and assembling of the circuit board assembly 360.

In an embodiment, the circuit board assembly 360 includes at least three groups of electrical connecting ends 363 for respectively achieving power supply, grounding, and signal line functions.

Preferably, each metal conductive area 341 is correspondingly provided with one group of electrical connecting ends 363 to achieve a corresponding function. In this embodiment, the circuit board is provided with three groups of electrical connecting ends 363, and each group includes two electrical connecting ends 363, that is, the circuit board is provided with three pairs of electrical connecting ends 363. The conductive ring 340 is provided with three metal conductive areas 341 and three conducting wires 343. The electrical connecting ends 363 rub against an outer side of the ring-shaped metal conductive areas 341, and when the conductive ring 340 rotates, the electrical connecting ends 363 are configured to maintain continuous electrical connection. By arranging the electrical connecting ends 363 in pairs, one is divided into two, so that when one is damaged, the other can still work. Further, the two electrical connecting ends 363 in each group are in contact with the same metal conductive area 341 from different directions to ensure the conducting effect.

It is to be understood that four or five groups of electrical connecting ends 363 may be provided according to design requirements or actual functional needs, and the number of electrical connecting ends 363 in each group may be set according to different current intensities.

Further, the electrical connecting ends 363 are configured as elastic members, and end portions of the elastic members are maintained in contact with the conductive ring 340 under an elastic action. In this way, even if wear occurs over time, the elastic members may remain in contact with the conductive ring 340 under the elastic action to maintain a good conducting effect.

In an embodiment, two circuit board assemblies 360 are provided and are clamped onto the bearing 350 to form a ring-shaped structure to be fitted to a periphery of the conductive ring 340.

Further, as shown in FIG. 14, the circuit board assemblies include a first circuit board assembly 361 and a second circuit board assembly 362. The first circuit board assembly 361 includes a first bracket 3611 and a first circuit board 3612 mounted on the first bracket 3611. The second circuit board assembly 362 includes a second bracket 3621 and a second circuit board 3622 mounted on the second bracket 3621. The first bracket 3611 and the second bracket 3621 both are of a semi-ring-shaped structure. The first bracket 3611 and the second bracket 3621 are combined to form a complete ring-shaped structure. The first circuit board 3612 and the second circuit board 3622 are respectively disposed in the front of the first bracket 3611 and the second bracket 3621. When the first circuit board assembly 361 and the second circuit board assembly 362 form a complete ring shape, the electrical connecting ends 363 on the first circuit board 3612 and the second circuit board 3622 are respectively in contact with the corresponding metal conductive areas 341 from two sides of the conductive ring 340 to achieve conduction. Specifically, in this embodiment, the first circuit board 3612 is provided with one group of electrical connecting ends 363, and the second circuit board 3622 is provided with two groups of electrical connecting ends 363. It is to be understood that the specific arrangement mode of the electrical connecting ends 363 may be adjusted according to actual functional design and use requirements.

Through this design, on the one hand, it is convenient to fix and mount the first circuit board 3612 and the second circuit board 3622, and at the same time, the circuit board assemblies 360 can be easily fitted onto or removed from the periphery of the conductive ring 340 without disassembling the conductive ring 340 itself; and on the other hand, the first bracket 3611 and the second bracket 3621 can also provide good protection and dust prevention for the conductive ring 340, thus further optimizing the conducting performance.

In an embodiment, two bearings 350 are provided and are respectively provided at two ends of the conductive ring 340, and the circuit board assembly 360 is clamped onto the two bearings 350.

Further, the apparatus for supplying power to the interior of the drum body of the drum washing machine further includes: an end cover 370, mounted at an end portion of the conductive ring 340 and configured to fix the inner ring of the bearing 350 from an outer side to ensure that the bearing 350 works normally. The end cover 370 is connected with the conductive ring 340 through a fastener.

In an embodiment, the functional assembly includes one or more of a gripping apparatus, a sterilization and drying apparatus, a position sensor, a temperature sensor, and a humidity sensor.

An embodiment of the present disclosure further provides a washing machine 300. As shown in FIG. 9 to FIG. 15, the washing machine includes the apparatus for supplying power to the interior of the drum body of the drum washing machine 300 described in Embodiment 1. When the washing machine 300 works, the tripod 330 drives the inner drum 310 to rotate under the driving of the driving motor; the conductive ring 340 fixed at the end portion of the rotating shaft 331 rotates together with the tripod 330 and the inner drum 310; the circuit board assembly 360 fixed on the outer ring of the bearing 350 of the rotating shaft 331 remains stationary relative to the outer tub 320; and during the rotation of the conductive ring 340, the electrical connecting ends 363 are in sliding contact with the conductive ring 340 to maintain a conducted state.

This embodiment achieves power supply to the functional assembly inside the inner drum 310 when the inner drum 310 rotates by arranging the conductive ring 340 and the circuit board assembly 360. In this way, the cooperation between the rotating inner drum 310 and the functional assembly can expand functions of the washing machine 300, optimize overall functions of the washing machine 300, meet different needs of users, and improve user experience. In addition, such arrangement can effectively avoid entanglement of conducting wires during rotation, the structure is simple, the operation is stable, and the reliability is high.

The present disclosure may be further described below in detail in combination with the specific implementations with reference to the drawings. It is to be noted that embodiments or technical features described below may be freely combined to form new embodiments without causing conflicts.

### Embodiment 4

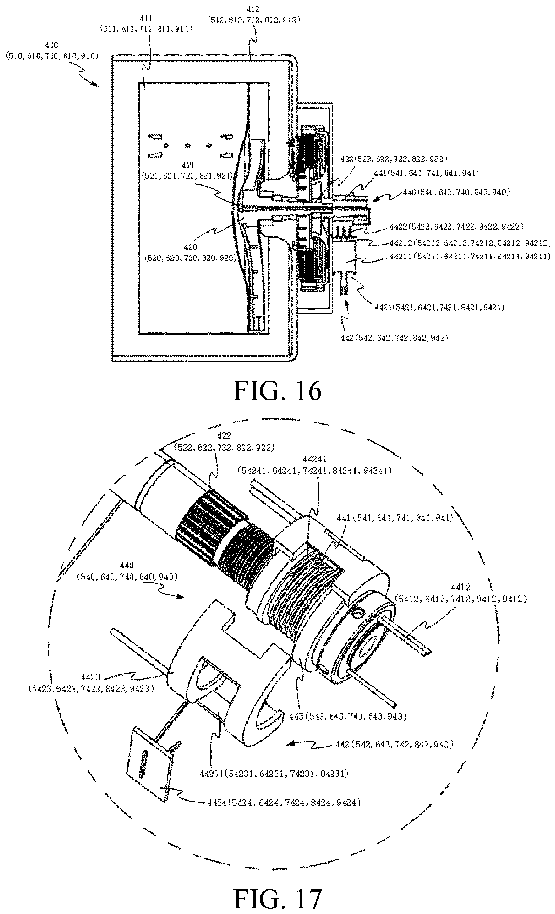

This embodiment provides an internal drum body 410 of a washing machine. As shown in FIG. 16 to FIG. 20, the internal drum body includes:
an inner drum 411, configured to accommodate clothes;
a tripod 420, fixedly connected with a bottom of the inner drum 411 to transmit a rotation driving force to the inner drum 411;
a power actuating mechanism 430, disposed inside the inner drum 411 to actuate a traction effect on the clothes; and
a power supply apparatus 440, located at one end of the tripod 420, where a wiring end of the power supply apparatus 440 penetrates through a hollow portion inside the tripod 420 and is connected with the power actuating mechanism 430.

The power supply apparatus 440 is configured to maintain power supply to the power actuating mechanism 430 in a process that the tripod 420 drives the inner drum 411 to rotate.

In this embodiment, the power supply apparatus 440 can supply power to the power actuating mechanism 430 mounted on the inner drum 411 when the inner drum 411 rotates. The power actuating mechanism 430 acts on the clothes in the inner drum 411 to increase washing power. For example, the clothes are driven to rotate back and forth to avoid entanglement, rubbing and tapping are strengthened, and the washing effect is improved. In this way, the cooperation between the rotating inner drum 411 and the power actuating mechanism 430 may further expand functions of the washing machine, optimize overall functions of the washing machine, meet different needs of users, and improve user experience. In addition, the arrangement that the wiring end of the power supply apparatus 440 penetrates through the hollow portion inside the tripod 420 can effectively avoid entanglement of conducting wires during rotation, the structure is simple, the operation is stable, and the reliability is high.

The power supply apparatus 440 is the apparatus for supplying power to the interior of the drum body of the drum washing machine in Embodiment 1 or Embodiment 3.

Further, the power supply apparatus 440 includes a conductive ring 441 and a fixed wiring portion 442 which are electrically connected.

The conductive ring 441 is fixed at an end portion of a rotating shaft 422 of the tripod 420 and synchronously rotates along with the tripod 420.

The fixed wiring portion 442 is mounted with a housing located outside the inner drum 411 and is configured to contact the conductive ring 441 to achieve electrical connection, so as to achieve electric conduction in a process that the conductive ring 441 rotates relative to the fixed wiring portion 442.

Further, the conductive ring 441 includes a plurality of metal conductive areas 4411 and a plurality of conducting wires 4412 corresponding to the metal conductive areas 4411.

An insulating plate 4413 is provided between adjacent metal conductive areas 4411.

One end of each conducting wire 4412 is electrically connected with the corresponding metal conductive area 4411, and the other end penetrates through a wiring passage 421 inside the tripod 420 to enter the inner drum 411 and connect with a power supply end of the power actuating mechanism 430.

In an embodiment, the fixed wiring portion 442 includes a telescopic electromagnet 4421 and a pogopin circuit board 4422. The telescopic electromagnet 4421 includes an electromagnet body 44211, a cylindrical protrusion, a spring, and a telescopic rod 44212. A center of the electromagnet body 44211 is provided with a mounting hole. One end of the telescopic rod 44212 penetrates through the mounting hole and is provided with the spring at the end portion, and the cylindrical protrusion is mounted at the other end of the telescopic rod. The cylindrical protrusion serves as an output end of the telescopic electromagnet 4421. The pogopin circuit board 4422 is mounted to the cylindrical protrusion.

The working flow of the telescopic electromagnet 4421 is as follows:
Power off: when it is not powered on, the telescopic electromagnet 4421 is in a non-working state, and the telescopic rod 44212 is in a non-popping-out state and is retracted in the electromagnet.
Power on: when a main control board of the washing machine is powered on, the telescopic electromagnet 4421 is in a working state, the telescopic rod 44212 is subjected to the magnetic force of the electromagnet, the magnetic force is greater than the elastic force of the spring, the telescopic rod 44212 pops out and maintains the set stroke distance.

It is to be noted that the fixed wiring portion 442 is of a telescopic structure. For different inner drum structures and actual design requirements of washing machines, the fixed wiring portion 442 may also be arranged as a swinging structure, a rotating structure, or a linear moving structure driven by a motor, an electric push rod, or any other driving apparatus. That is, the solution that can achieve contact and separation between the conductive ring 441 and the fixed wiring portion 442 is within the scope of protection of the present disclosure.

In another embodiment, the fixed wiring portion 442 includes a bracket 4423 and a circuit board 4424 fixed to the bracket 4423.

The bracket 4423 is rotatably mounted with the conductive ring 441 through a bearing 443, and an opening 44231 is formed in a sidewall of the bracket 4423.

The circuit board 4424 is mounted in the opening 44231, and electrical connecting ends 44241 are formed on one side of the circuit board 4424 towards the conductive ring 441, and are always in contact with a sidewall of the conductive ring 441 to achieve electric conduction in a process that the conductive ring 441 rotates relative to the bracket 4423.

In an embodiment, the power actuating mechanism 430 includes one or more of a gripping apparatus, a poking apparatus, a stirring apparatus, and a lifting apparatus. Specifically, the gripping apparatus can grip and unfold the clothes to increase a contact area between washing water and the clothes, or facilitate rapid drying of the clothes in the drying process, the poking apparatus or stirring apparatus can increase a rubbing effect on the clothes, and the lifting apparatus can increase a tapping effect on the clothes to avoid clumping, thus effectively improving the washing cleanliness of the washing machine and achieving diversified washing modes.

In an embodiment, the power actuating mechanism 430 is mounted on a sidewall of the inner drum 411.

Further, the power actuating mechanism 430 is mounted on an inner sidewall of the inner drum 411. For example, the gripping apparatus and the poking apparatus that facilitate providing power to the items in the inner drum 411 during rotation or non-rotation may be provided, so as to accelerate washing.

Further, the power actuating mechanism 430 is distributed on an outer sidewall of the inner drum 411. For example, a cleaning apparatus that can serve as the outer tub 412 may be arranged on an outer wall of the inner drum 411. Specifically, the cleaning apparatus includes a retractable cleaning brush. In the cleaning process, the cleaning brush is controlled to extend and make bristles contact the inner wall of the outer tub 412 to clean the inner wall of the outer tub 412 when the inner drum 411 rotates. When the cleaning brush is reset, the cleaning process ends.

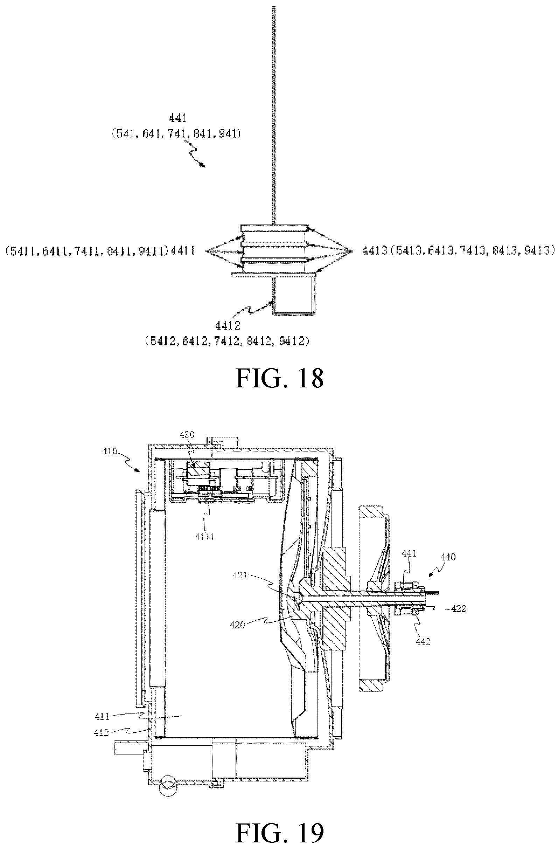

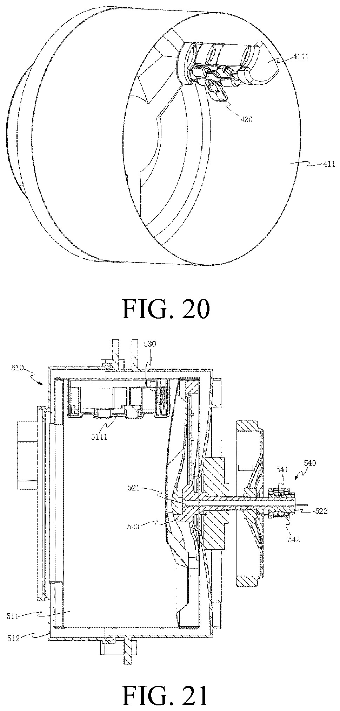

In an embodiment, as shown in FIG. 19 and FIG. 20, the inner drum 411 further includes a lifter 4111, and the power actuating mechanism 430 is mounted with the lifter 4111. Specifically, the power actuating mechanism 430 may be mounted inside or outside the lifter 4111. During the rotation of the inner drum 411, the traction effect of the power actuating mechanism 430 cooperates with the lifter 4111 to achieve lifting and tapping effects on the clothes, thus further improving the cleaning effect on the clothes.

Further, the power actuating mechanism 430 is mounted inside the lifter 4111, and after the work is completed, the power actuating mechanism 430 is hidden inside the lifter 4111. Specifically, the lifter 4111 is provided with a mounting port, the power actuating mechanism 430 is mounted inside the lifter 4111, and an execution end of the power actuating mechanism extends from the lifter 4111 through the mounting port to act on the clothes. The arrangement that the power actuating mechanism 430 is hidden inside the lifter 4111 optimizes the structural layout of the inner drum 411, and is conducive to the attractiveness of the inner drum 411.

In another embodiment, the power actuating mechanism 430 is mounted at the bottom of the inner drum 411 to provide power acting on the items at an inner side of the bottom of the inner drum during the rotation or non-rotation of the inner drum 411. For example, a gripping apparatus and a poking apparatus can play the role of shaking and dispersing the clothes at the bottom of the drum and preventing them from clumping at the bottom of the drum in the washing process, so as to accelerate the washing process. A cleaning apparatus that can serve as the inner wall of the bottom of the outer tub 412 may also be provided on the outer wall of the bottom of the inner drum 411.

A washing machine includes the internal drum body 410 of the washing machine described above. In this embodiment, the power supply apparatus 440 can supply power to the power actuating mechanism 430 mounted on the inner drum 411 when the inner drum 411 rotates. The power actuating mechanism 430 acts on the clothes in the inner drum 411 to increase washing power. For example, the clothes are driven to rotate back and forth to avoid entanglement, rubbing and tapping are strengthened, and the washing effect is improved. In this way, the cooperation between the rotating inner drum 411 and the power actuating mechanism 430 may further expand functions of the washing machine, optimize overall functions of the washing machine, meet different needs of users, and improve user experience. In addition, the arrangement that the wiring end of the power supply apparatus 440 penetrates through the hollow portion inside the tripod 420 can effectively avoid entanglement of conducting wires during rotation, the structure is simple, the operation is stable, and the reliability is high. The power actuating mechanism 430 is the apparatus for supplying power to the interior of the drum body of the drum washing machine described in Embodiment 1 or Embodiment 3.

### Embodiment 5

An embodiment of the present disclosure provides an internal drum body 510 of a washing machine. As shown in FIG. 16 to FIG. 18 and FIG. 21 to FIG. 23, the internal drum body includes:
an inner drum 511, configured to accommodate clothes;
a tripod 520, fixedly connected with a bottom of the inner drum 511 to transmit a rotation driving force to the inner drum 511;
a sensing apparatus 530, disposed in the inner drum 511 to sense environmental parameters in the inner drum; and
a power supply apparatus 540, located at one end of the tripod 520, where a wiring end of the power supply apparatus 540 penetrates through a hollow portion inside the tripod 520 and is connected with the sensing apparatus 530.

The power supply apparatus 540 is configured to maintain power supply to the sensing apparatus 530 in a process that the tripod 520 drives the inner drum 511 to rotate.

In this embodiment, the power supply apparatus 540 can supply power to the sensing apparatus 530 mounted on the inner drum 511 when the inner drum 511 rotates, and the sensing apparatus 530 detects and feeds back the washing environment inside the drum for real-time adjustment of the washing mode. In this way, the cooperation between the rotating inner drum 511 and the sensing apparatus 530 may further expand functions of the washing machine, optimize overall functions of the washing machine, meet different needs of users, and improve user experience. In addition, the arrangement that the wiring end of the power supply apparatus 540 penetrates through the hollow portion inside the tripod 520 can effectively avoid entanglement of conducting wires during rotation, the structure is simple, the operation is stable, and the reliability is high.

The power supply apparatus 540 is the apparatus for supplying power to the interior of the drum body of the drum washing machine in Embodiment 1 or Embodiment 3.

Further, the power supply apparatus 540 includes a conductive ring 541 and a fixed wiring portion 542 which are electrically connected.

The conductive ring 541 is fixed at an end portion of a rotating shaft 522 of the tripod 520 and synchronously rotates along with the tripod 520.

The fixed wiring portion 542 is mounted with a housing located outside the inner drum 511 and is configured to contact the conductive ring 541 to achieve electrical connection, so as to achieve electric conduction in a process that the conductive ring 541 rotates relative to the fixed wiring portion 542.

Further, the conductive ring 541 includes a plurality of metal conductive areas 5411 and a plurality of conducting wires 5412 corresponding to the metal conductive areas 5411.

An insulating plate 5413 is provided between adjacent metal conductive areas 5411.

One end of each conducting wire 5412 is electrically connected with the corresponding metal conductive area 5411, and the other end penetrates through a wiring passage 521 inside the tripod 520 to enter the inner drum 511 and connect with a power supply end of the sensing apparatus 530.

In an embodiment, the fixed wiring portion 542 includes a telescopic electromagnet 5421 and a pogopin circuit board 5422. The telescopic electromagnet 5421 includes an electromagnet body 54211, a cylindrical protrusion, a spring, and a telescopic rod 54212. A center of the electromagnet body 54211 is provided with a mounting hole. One end of the telescopic rod 54212 penetrates through the mounting hole and is provided with the spring at the end portion, and the cylindrical protrusion is mounted at the other end of the telescopic rod. The cylindrical protrusion serves as an output end of the telescopic electromagnet 5421. The pogopin circuit board 5422 is mounted to the cylindrical protrusion.

The working flow of the telescopic electromagnet 5421 is as follows:
Power off: when it is not powered on, the telescopic electromagnet 5421 is in a non-working state, and the telescopic rod 54212 is in a non-popping-out state and is retracted in the electromagnet.
Power on: when a main control board of the washing machine is powered on, the telescopic electromagnet 5421 is in a working state, the telescopic rod 54212 is subjected to the magnetic force of the electromagnet, the magnetic force is greater than the elastic force of the spring, the telescopic rod 54212 pops out and maintains the set stroke distance.

It is to be noted that the fixed wiring portion 542 is of a telescopic structure. For different inner drum structures and actual design requirements of washing machines, the fixed wiring portion 542 may also be arranged as a swinging structure, a rotating structure, or a linear moving structure driven by a motor, an electric push rod, or any other driving apparatus. That is, the solution that can achieve contact and separation between the conductive ring 541 and the fixed wiring portion 542 is within the scope of protection of the present disclosure.

In another embodiment, the fixed wiring portion 542 includes a bracket 5423 and a circuit board 5424 fixed to the bracket 5423.

The bracket 5423 is rotatably mounted with the conductive ring 541 through a bearing 543, and an opening 54231 is formed in a sidewall of the bracket 5423.

The circuit board 5424 is mounted in the opening 54231, and electrical connecting ends 54241 are formed on one side of the circuit board 5424 towards the conductive ring 541, and are always in contact with a sidewall of the conductive ring 541 to achieve electric conduction in a process that the conductive ring 541 rotates relative to the bracket 5423.

The environmental parameters include parameters such as water level, water temperature, and water quality. Further, the sensing apparatus 530 includes one or more of a water level sensor, a temperature sensor, a humidity sensor, an acceleration sensor, a position sensor, a weight sensor, and a water quality sensor.

The water level sensor is configured to detect the level of water in the washing machine to control the filling and discharging of water.

The temperature sensor is configured to detect the temperature of water in the washing machine during the rotation of the inner drum 511 to ensure that the temperature of water in the washing machine reaches set temperature.

The humidity sensor is configured to detect the humidity in the washing machine during the rotation of the inner drum 511 to ensure that the humidity in the washing machine reaches set humidity.

The acceleration sensor is configured to detect movement parameters of the drum body during the rotation of the inner drum 511, such as rotating speed and rotating acceleration.

The position sensor is configured to detect a relative or absolute position of a specific structure or apparatus during the rotation of the inner drum 511, which is used as a condition input to trigger a specific execution program.

The weight sensor is configured to adjust the water level in the washing machine and the rotating speed by detecting the weight in the washing drum during the rotation of the inner drum 511, so as to control the operation of the washing program and achieve the effect of saving water and electricity.

The water quality sensor is configured to sense dissolved substances, impurities, and stains in the washing water during the rotation of the inner drum 511 and feed back to the main control board of the washing machine. The water quality sensor includes a turbidity sensor, a TOC sensor, a TDS sensor, and the like.

**In** an embodiment, the sensing apparatus 530 is mounted on a sidewall of the inner drum 511. The sensing apparatus 530 may be mounted on the inner wall of the inner drum 511 or the outer wall of the inner drum 511 to sense the environmental parameters in the drum during rotation or non-rotation of the inner drum 511. For example, the temperature sensor may be mounted on the inner wall or outer wall of the inner drum 511 to more accurately determine the temperature in the drum body 510, including water temperature or drying temperature. The weight sensor may be mounted on the inner wall or outer wall of the inner drum 511 to more accurately sense the weight of the items in the drum body 510. When the water quality sensor is mounted on the inner wall of the inner drum 511, the extent of turbidity of the washing water in the drum body 510 may be detected. When the water quality sensor is mounted on the outer wall of the inner drum 511, the extent of turbidity of the washing water flowing out of the inner drum 511 or the extent of turbidity of the washing water in a space sandwiched between the inner drum 511 and the outer tub 512.

**In** an embodiment, as shown in FIG. 21 to FIG. 23, the inner drum 511 further includes a lifter 5111, and the sensing apparatus 530 is fixedly mounted with the lifter 5111.

Further, the sensing apparatus 530 includes at least one sensing area 531, such as a sensing probe, and the at least one sensing area is embedded into a surface of the lifter 5111. Specifically, the sensing apparatus 530 includes a sensing circuit board 532 and a sensing area 531 connected with the sensing circuit board 532. The sensing circuit board 532 is clamped inside the lifter 5111 and connected with the main control board of the washing machine. The sensing area 531 extends from the surface of the lifter 5111 to detect and sense the environmental parameters in the drum.

The arrangement that the sensing apparatus 530 is hidden inside the lifter 5111 optimizes the structural layout of the inner drum 511, and is conducive to the attractiveness of the inner drum 511. At the same time, the lifter 5111 may form an outer shell structure of the sensing apparatus 530 to provide protection.

Further, the surface of the lifter 5111 is provided with a through hole 51111, and the sensing area 531 is mounted in the through hole 51111. Specifically, the shape of the through hole 51111 adapts to the shape of the sensing area 531, making it easy to mount while maintaining good stability.

**In** an embodiment, the sensing apparatus 530 is mounted at the bottom of the inner drum 511. For example, to simplify the detection method and improve the detection accuracy, the water level sensor is mounted at a center position of the bottom of the inner drum 511 to ensure real-time detection of the water level in the drum in a process that the inner drum 511 rotates.

A washing machine includes the internal drum body 510 of the washing machine described above. **In** this embodiment, the power supply apparatus 540 can supply power to the sensing apparatus 530 mounted on the inner drum 511 when the inner drum 511 rotates, and the sensing apparatus 530 detects and feeds back the washing environment inside the drum for real-time adjustment of the washing mode. **In** this way, the cooperation between the rotating inner drum 511 and the sensing apparatus 530 may further expand functions of the washing machine, optimize overall functions of the washing machine, meet different needs of users, and improve user experience. In addition, the arrangement that the wiring end of the power supply apparatus 540 penetrates through the hollow portion inside the tripod 520 can effectively avoid entanglement of conducting wires during rotation, the structure is simple, the operation is stable, and the reliability is high. The power supply apparatus 540 is the apparatus for supplying power to the interior of the drum body of the drum washing machine in Embodiment 1 or Embodiment 3.

### Embodiment 6

This embodiment provides an internal drum body 610 of a washing machine. As shown in FIG. 16 to FIG. 18 and FIG. 24 to FIG. 25, the internal drum body includes:
an inner drum 611, configured to accommodate clothes;
a tripod 620, fixedly connected with a bottom of the inner drum 611 to transmit a rotation driving force to the inner drum 611;
a heating apparatus 630, disposed in the inner drum 611 to provide a thermal environment of the inner drum body 610; and
a power supply apparatus 640, located at one end of the tripod 620, where a wiring end of the power supply apparatus 640 penetrates through a hollow portion inside the tripod 620 and is connected with the heating apparatus 630.

The power supply apparatus 640 is configured to maintain power supply to the heating apparatus 630 in a process that the tripod 620 drives the inner drum 611 to rotate.

In this embodiment, the power supply apparatus 640 can supply power to the heating apparatus 630 mounted on the inner drum 611 when the inner drum 611 rotates, and the heating apparatus 630 heats the environment inside the inner drum 611 to improve the washing effect. In this way, the cooperation between the rotating inner drum 611 and the heating apparatus 630 may further expand functions of the washing machine, optimize overall functions of the washing machine, meet different needs of users, and improve user experience. In addition, the arrangement that the wiring end of the power supply apparatus 640 penetrates through the hollow portion inside the tripod 620 can effectively avoid entanglement of conducting wires during rotation, the structure is simple, the operation is stable, and the reliability is high.

The power supply apparatus 640 is the apparatus for supplying power to the interior of the drum body of the drum washing machine in Embodiment 1 or Embodiment 3.

Further, the power supply apparatus 640 includes a conductive ring 641 and a fixed wiring portion 642 which are electrically connected.

The conductive ring 641 is fixed at an end portion of a rotating shaft 622 of the tripod 620 and synchronously rotates along with the tripod 620.

The fixed wiring portion 642 is mounted with a housing located outside the inner drum 611 and is configured to contact the conductive ring
641 to achieve electrical connection, so as to achieve electric conduction in a process that the conductive ring 641 rotates relative to the fixed wiring portion 642.

Further, the conductive ring 641 includes a plurality of metal conductive areas 6411 and a plurality of conducting wires 6412 corresponding to the metal conductive areas 6411.

An insulating plate 6413 is provided between adjacent metal conductive areas 6411.

One end of each conducting wire 6412 is electrically connected with the corresponding metal conductive area 6411, and the other end penetrates through a wiring passage 621 inside the tripod 620 to enter the inner drum 611 and connect with a power supply end of the heating apparatus 630.

**In** an embodiment, the fixed wiring portion 642 includes a telescopic electromagnet 6421 and a pogopin circuit board 6422. The telescopic electromagnet 6421 includes an electromagnet body 64211, a cylindrical protrusion, a spring, and a telescopic rod 64212. A center of the electromagnet body 64211 is provided with a mounting hole. One end of the telescopic rod 64212 penetrates through the mounting hole and is provided with the spring at the end portion, and the cylindrical protrusion is mounted at the other end of the telescopic rod. The cylindrical protrusion serves as an output end of the telescopic electromagnet 6421. The pogopin circuit board 6422 is mounted to the cylindrical protrusion.

The working flow of the telescopic electromagnet 6421 is as follows:
Power off: when it is not powered on, the telescopic electromagnet 6421 is in a non-working state, and the telescopic rod 64212 is in a non-popping-out state and is retracted in the electromagnet.
Power on: when a main control board of the washing machine is powered on, the telescopic electromagnet 6421 is in a working state, the telescopic rod 64212 is subjected to the magnetic force of the electromagnet, the magnetic force is greater than the elastic force of the spring, the telescopic rod 64212 pops out and maintains the set stroke distance.

It is to be noted that the fixed wiring portion 642 is of a telescopic structure. For different inner drum structures and actual design requirements of washing machines, the fixed wiring portion 642 may also be arranged as a swinging structure, a rotating structure, or a linear moving structure driven by a motor, an electric push rod, or any other driving apparatus. That is, the solution that can achieve contact and separation between the conductive ring 641 and the fixed wiring portion 642 is within the scope of protection of the present disclosure.

**In** another embodiment, the fixed wiring portion 642 includes a bracket 6423 and a circuit board 6424 fixed to the bracket 6423.

The bracket 6423 is rotatably mounted with the conductive ring 641 through a bearing 643, and an opening 64231 is formed in a sidewall of the bracket 6423.

The circuit board 6424 is mounted in the opening 64231, and electrical connecting ends 64241 are formed on one side of the circuit board 4424 towards the conductive ring 641, and are always in contact with a sidewall of the conductive ring 641 to achieve electric conduction in a process that the conductive ring 641 rotates relative to the bracket 6423.

In an embodiment, the heating apparatus 630 is mounted on a sidewall of the inner drum 611. The heating apparatus 630 may be mounted on the inner wall of the inner drum 611 or the outer wall of the inner drum 611 to provide a heating function during rotation or non-rotation of the inner drum 611. Specifically, for example, when the heating apparatus 630 is mounted on the inner wall of the inner drum 611, the washing water inside the inner drum 611 may be directly heated; and when the heating apparatus 630 is mounted on the outer wall of the inner drum 611, the washing water in a space sandwiched between the inner drum 611 and the outer tub 612 may be heated, and the heated washing water cooperates with the rotation of the inner drum 611 to further improve the cleaning effect on the clothes.

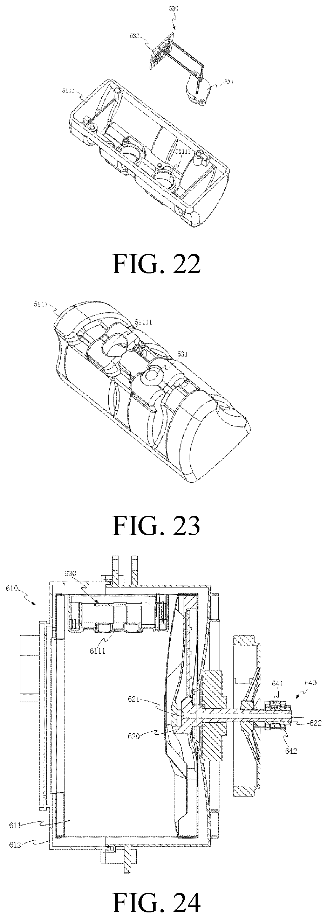

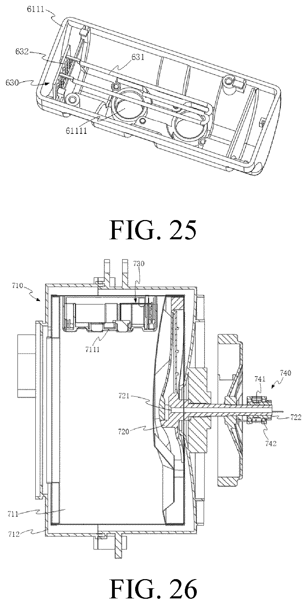

In an embodiment, as shown in FIG. 24 and FIG. 25, the inner drum 611 further includes a lifter 6111, and the heating apparatus 630 is mounted in the lifter 6111. Specifically, the lifter 6111 is provided with a water hole 61111. The washing water enters the lifter 6111 through the water hole 61111, is heated by the heating apparatus 630, and then flows out through the water hole 61111 to clean the clothes. On the one hand, the arrangement that the heating apparatus 630 is hidden inside the lifter 6111 optimizes the structural layout of the inner drum 611, and is conducive to the attractiveness of the inner drum 611. On the other hand, the heating effect of the heating apparatus 630 cooperates with the smashing and stirring effect of the lifter 6111 on the clothes, thus optimizing the washing effect.

Further, the heating apparatus 630 includes a heating tube 631, and the heating tube extends along a length direction of the lifter 6111. Specifically, the heating apparatus 630 includes a connecting portion 632 and a heating tube 631. The connecting portion 632 is mounted at one end of the inner wall of the lifter 6111 and is electrically connected with the wiring end of the power supply apparatus 640. The heating tube 631 is mounted on one side of the connecting portion 632 and extends along the length direction of the lifter 6111 to the other end of the lifter 6111. This arrangement is conducive to increasing a heating area, improving a contact area between the washing water and the heating tube, thus improving heating efficiency and optimizing the washing effect. It is to be noted that the heating apparatus 630 may also be configured as a heating sheet or PTC heating ceramic.

**In** an embodiment, the heating apparatus 630 is mounted at the bottom of the inner drum 611. The heating apparatus 630 may be mounted on the inner wall of the bottom of the drum or the outer wall of the bottom of the drum to provide a heating function at the bottom of the drum during rotation or non-rotation of the inner drum 611. Further, the heating apparatus 630 may also be mounted in the tripod 620.

**In** an embodiment, the heating apparatus 630 is arranged close to the tripod 620 to facilitate the layout of wiring harness.

This embodiment provides a washing machine, which includes the internal drum body 610 of the washing machine described above. **In** this embodiment, the power supply apparatus 640 can supply power to the heating apparatus 630 mounted on the inner drum 611 when the inner drum 611 rotates, and the heating apparatus 630 heats the environment inside the inner drum 611 to improve the washing effect. **In** this way, the cooperation between the rotating inner drum 611 and the heating apparatus 630 may further expand functions of the washing machine, optimize overall functions of the washing machine, meet different needs of users, and improve user experience. **In** addition, the arrangement that the wiring end of the power supply apparatus 640 penetrates through the hollow portion inside the tripod 620 can effectively avoid entanglement of conducting wires during rotation, the structure is simple, the operation is stable, and the reliability is high. The heating apparatus 630 is the apparatus for supplying power to the interior of the drum body of the drum washing machine in Embodiment 1 or Embodiment 3.

An embodiment of the present disclosure provides an internal drum body of a washing machine, which includes:
an inner drum, configured to accommodate clothes;
a tripod, fixedly connected with a bottom of the inner drum to transmit a rotation driving force to the inner drum;
a sterilization apparatus, disposed inside the inner drum to perform sterilization and purification on the drum body; and
a power supply apparatus, located at one end of the tripod, where a wiring end of the power supply apparatus penetrates through a hollow portion inside the tripod and is connected with the sterilization apparatus.

The power supply apparatus is configured to maintain power supply to the sterilization apparatus in a process that the tripod drives the inner drum to rotate.

The sterilization apparatus includes a light sterilization apparatus, a hypochlorous acid sterilization apparatus, and a high-temperature steam sterilization apparatus, which respectively correspond to Embodiment 7, Embodiment 8, and Embodiment 9. It is to be noted that the light sterilization apparatus includes a UV sterilization apparatus or a light plasma sterilization apparatus. In Embodiment 7, description is made by taking a UV sterilization apparatus as an example. Alternatively, the UV sterilization apparatus 730 may be replaced with a light plasma sterilization apparatus. By using the effect of high-voltage plasma discharge, air, water, and harmful substance molecules in the inner drum are decomposed into highly active ions such as oxygen ions, negative ions, and free radicals, thus killing bacteria, viruses, and other microorganisms in the inner drum. In Embodiment 9, the steam generation apparatus can not only sterilize the environment inside the drum to form the high-temperature steam sterilization apparatus, but also perform steam cleaning or steam care on the clothes inside the drum.

### Embodiment 7

This embodiment of the present disclosure further provides an internal drum body 710 of a washing machine. As shown in FIG. 16 to FIG. 18 and FIG. 26 to FIG. 28, the internal drum body includes:
an inner drum 711, configured to accommodate clothes;
a tripod 720, fixedly connected with a bottom of the inner drum 711 to transmit a rotation driving force to the inner drum 711;
a UV sterilization apparatus 730, disposed inside the inner drum 711 to perform sterilization and purification on the drum body 710; and
a power supply apparatus 740, located at one end of the tripod 720, where a wiring end of the power supply apparatus 740 penetrates through a hollow portion inside the tripod 720 and is connected with the UV sterilization apparatus 730.

The power supply apparatus 740 is configured to maintain power supply to the UV sterilization apparatus 730 in a process that the tripod 720 drives the inner drum 711 to rotate.

In this embodiment, the power supply apparatus 740 can supply power to the UV sterilization apparatus 730 mounted on the inner drum 711 when the inner drum 711 rotates, and the UV sterilization apparatus 730 can sterilize the environment inside the drum, including the clothes inside the drum when the inner drum 711 rotates, so as to improve the cleanliness of the drum body 710 and the clothes. In this way, the cooperation between the rotating inner drum 711 and the UV sterilization apparatus 730 may further expand functions of the washing machine, optimize overall functions of the washing machine, meet different needs of users, and improve user experience. In addition, the arrangement that the wiring end of the power supply apparatus 740 penetrates through the hollow portion inside the tripod 720 can effectively avoid entanglement of conducting wires during rotation, the structure is simple, the operation is stable, and the reliability is high.

The power supply apparatus 740 is the apparatus for supplying power to the interior of the drum body of the drum washing machine in Embodiment 1 or Embodiment 3.

Further, the power supply apparatus 740 includes a conductive ring 741 and a fixed wiring portion 742 which are electrically connected.

The conductive ring 741 is fixed at an end portion of a rotating shaft 722 of the tripod 720 and synchronously rotates along with the tripod 720.

The fixed wiring portion 742 is mounted with a housing located outside the inner drum 711 and is configured to contact the conductive ring 741 to achieve electrical connection, so as to achieve electric conduction in a process that the conductive ring 741 rotates relative to the fixed wiring portion 742.

Further, the conductive ring 741 includes a plurality of metal conductive areas 7411 and a plurality of conducting wires 7412 corresponding to the metal conductive areas 7411.

An insulating plate 7413 is provided between adjacent metal conductive areas 7411.

One end of each conducting wire 7412 is electrically connected with the corresponding metal conductive area 7411, and the other end penetrates through a wiring passage 721 inside the tripod 720 to enter the inner drum 711 and connect with a power supply end of the UV sterilization apparatus 730.

In an embodiment, the fixed wiring portion 742 includes a telescopic electromagnet 7421 and a pogopin circuit board 7422. The telescopic electromagnet 7421 includes an electromagnet body 74211, a cylindrical protrusion, a spring, and a telescopic rod 74212. A center of the electromagnet body 74211 is provided with a mounting hole. One end of the telescopic rod 74212 penetrates through the mounting hole and is provided with the spring at the end portion, and the cylindrical protrusion is mounted at the other end of the telescopic rod. The cylindrical protrusion serves as an output end of the telescopic electromagnet 7421. The pogopin circuit board 7422 is mounted to the cylindrical protrusion.

The working flow of the telescopic electromagnet 7421 is as follows:
Power off: when it is not powered on, the telescopic electromagnet 7421 is in a non-working state, and the telescopic rod 74212 is in a non-popping-out state and is retracted in the electromagnet.
Power on: when a main control board of the washing machine is powered on, the telescopic electromagnet 7421 is in a working state, the telescopic rod 74212 is subjected to the magnetic force of the electromagnet, the magnetic force is greater than the elastic force of the spring, the telescopic rod 74212 pops out and maintains the set stroke distance.

It is to be noted that the fixed wiring portion 742 is of a telescopic structure. For different inner drum structures and actual design requirements of washing machines, the fixed wiring portion 742 may also be arranged as a swinging structure, a rotating structure, or a linear moving structure driven by a motor, an electric push rod, or any other driving apparatus. That is, the solution that can achieve contact and separation between the conductive ring 741 and the fixed wiring portion 742 is within the scope of protection of the present disclosure.

In another embodiment, the fixed wiring portion 742 includes a bracket 7423 and a circuit board 7424 fixed to the bracket 7423.

The bracket 7423 is rotatably mounted with the conductive ring 741 through a bearing 743, and an opening 74231 is formed in a sidewall of the bracket 7423.

The circuit board 7424 is mounted in the opening 74231, and electrical connecting ends 74241 are formed on one side of the circuit board 7424 towards the conductive ring 741, and are always in contact with a sidewall of the conductive ring 741 to achieve electric conduction in a process that the conductive ring 741 rotates relative to the bracket 7423.

In an embodiment, the UV sterilization apparatus 730 is mounted on the sidewall of the inner drum 711 to provide a sterilization function to the drum body 710 during the rotation or non-rotation of the inner drum 711.

Further, the UV sterilization apparatus 730 is mounted on an inner sidewall of the inner drum 711 to sterilize the clothes and washing water in the drum.

Further, the UV sterilization apparatus 730 is mounted on an outer sidewall of the inner drum 711 to sterilize a space sandwiched between the inner drum 711 and the outer tub 712.

In an embodiment, as shown in FIG. 26, FIG. 27 and FIG. 28, the inner drum 711 further includes a lifter 7111, and the UV sterilization apparatus 730 is fixedly mounted with the lifter 7111.

Further, the UV sterilization apparatus 730 includes at least one UV lamp holder 731, and the at least one UV lamp holder is embedded into a surface of the lifter 7111. Specifically, the UV sterilization apparatus 730 includes a UV circuit board 732 and a UV lamp holder 731 connected with the UV circuit board 732. The UV circuit board 732 is clamped inside the lifter 7111 and connected with the main control board of the washing machine. The UV lamp holder 731 extends from the surface of the lifter 7111 to perform sterilization and purification on the environment in the drum.

The arrangement that the UV sterilization apparatus 730 is hidden inside the lifter 7111 optimizes the structural layout of the inner drum 711, and is conducive to the attractiveness of the inner drum 711. At the same time, the lifter 7111 may form an outer shell structure of the UV sterilization apparatus 730 to provide protection.

Further, the surface of the lifter 7111 is provided with a through hole 71111, and the UV lamp holder 731 is mounted in the through hole 71111. Specifically, the shape of the through hole 71111 adapts to the shape of the UV lamp holder 731, making it easy to mount while maintaining good stability.

In an embodiment, the UV sterilization apparatus 730 is mounted at the bottom of the inner drum 711. Specifically, the UV sterilization apparatus 730 may be mounted at the inner side or outer side of the bottom of the drum. When it is mounted at the inner side, the environment and clothes in the inner drum 711 may be sterilized. When it is mounted at the outer side, the outer wall of the bottom of the inner drum 711 and the inner wall of the bottom of the outer tub 712 may be sterilized.

A washing machine includes the internal drum body 710 of the washing machine described above. In this embodiment, the power supply apparatus 740 can supply power to the UV sterilization apparatus 730 mounted on the inner drum 711 when the inner drum 711 rotates, and the UV sterilization apparatus 730 can sterilize the environment inside the drum, including the clothes inside the drum when the inner drum 711 rotates, so as to improve the cleanliness of the drum body 710 and the clothes. In this way, the cooperation between the rotating inner drum 711 and the UV sterilization apparatus 730 may further expand functions of the washing machine, optimize overall functions of the washing machine, meet different needs of users, and improve user experience. In addition, the arrangement that the wiring end of the power supply apparatus 740 penetrates through the hollow portion inside the tripod 720 can effectively avoid entanglement of conducting wires during rotation, the structure is simple, the operation is stable, and the reliability is high. The power supply apparatus 740 is the apparatus for supplying power to the interior of the drum body of the drum washing machine in Embodiment 1 or Embodiment 3.

### Embodiment 8

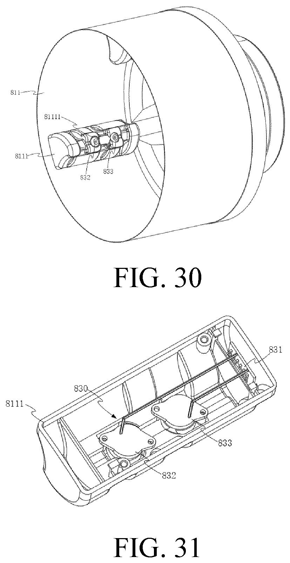

This embodiment provides an internal drum body 810 of a washing machine. As shown in FIG. 16 to FIG. 18 and FIG. 29 to FIG. 31, the internal drum body includes:
an inner drum 811, configured to accommodate clothes;
a tripod 820, fixedly connected with a bottom of the inner drum 811 to transmit a rotation driving force to the inner drum 811;
a hypochlorous acid sterilization apparatus 830, disposed in the inner drum 811 to perform sterilization and purification on the drum body 810; and
a power supply apparatus 840, located at one end of the tripod 820, where a wiring end of the power supply apparatus 840 penetrates through a hollow portion inside the tripod 820 and is connected with the hypochlorous acid sterilization apparatus 830.

The power supply apparatus 840 is configured to maintain power supply to the hypochlorous acid sterilization apparatus 830 in a process that the tripod 820 drives the inner drum 811 to rotate.

**In** this embodiment, the power supply apparatus 840 can supply power to the hypochlorous acid sterilization apparatus 830 mounted on the inner drum 811 when the inner drum 811 rotates, water is electrolyzed by the hypochlorous acid sterilization apparatus 830, then slightly acidic electrolyzed water is generated. The slightly acidic electrolyzed water has strong oxidation ability and fast and efficient microbial killing effect, and can sterilize the environment inside the drum, including the clothes inside the drum when the inner drum 811 rotates, so as to improve the cleanliness of the drum body 810 and the clothes, and achieve high safety. **In** this way, the cooperation between the rotating inner drum 811 and the hypochlorous acid sterilization apparatus 830 may further expand functions of the washing machine, optimize overall functions of the washing machine, meet different needs of users, and improve user experience. **In** addition, the arrangement that the wiring end of the power supply apparatus 840 penetrates through the hollow portion inside the tripod 820 can effectively avoid entanglement of conducting wires during rotation, the structure is simple, the operation is stable, and the reliability is high.

The power supply apparatus 840 is the apparatus for supplying power to the interior of the drum body of the drum washing machine in Embodiment 1 or Embodiment 3.

Further, the power supply apparatus 840 includes a conductive ring 841 and a fixed wiring portion 842 which are electrically connected.

The conductive ring 841 is fixed at an end portion of a rotating shaft 822 of the tripod 820 and synchronously rotates along with the tripod 820.

The fixed wiring portion 842 is mounted with a housing located outside the inner drum 811 and is configured to contact the conductive ring 841 to achieve electrical connection, so as to achieve electric conduction in a process that the conductive ring 841 rotates relative to the fixed wiring portion 842.

Further, the conductive ring 841 includes a plurality of metal conductive areas 8411 and a plurality of conducting wires 8412 corresponding to the metal conductive areas 8411.

An insulating plate 8413 is provided between adjacent metal conductive areas 8411.

One end of each conducting wire 8412 is electrically connected with the corresponding metal conductive area 8411, and the other end penetrates through a wiring passage 821 inside the tripod 820 to enter the inner drum 811 and connect with a power supply end of the hypochlorous acid sterilization apparatus 830.

In an embodiment, the fixed wiring portion 842 includes a telescopic electromagnet 8421 and a pogopin circuit board 8422. The telescopic electromagnet 8421 includes an electromagnet body 84211, a cylindrical protrusion, a spring, and a telescopic rod 84212. A center of the electromagnet body 84211 is provided with a mounting hole. One end of the telescopic rod 84212 penetrates through the mounting hole and is provided with the spring at the end portion, and the cylindrical protrusion is mounted at the other end of the telescopic rod. The cylindrical protrusion serves as an output end of the telescopic electromagnet 8421. The pogopin circuit board 8422 is mounted to the cylindrical protrusion.

The working flow of the telescopic electromagnet 8421 is as follows:
Power off: when it is not powered on, the telescopic electromagnet 8421 is in a non-working state, and the telescopic rod 84212 is in a non-popping-out state and is retracted in the electromagnet.
Power on: when a main control board of the washing machine is powered on, the telescopic electromagnet 8421 is in a working state, the telescopic rod 84212 is subjected to the magnetic force of the electromagnet, the magnetic force is greater than the elastic force of the spring, the telescopic rod 84212 pops out and maintains the set stroke distance.

It is to be noted that the fixed wiring portion 842 is of a telescopic structure. For different inner drum structures and actual design requirements of washing machines, the fixed wiring portion 842 may also be arranged as a swinging structure, a rotating structure, or a linear moving structure driven by a motor, an electric push rod, or any other driving apparatus. That is, the solution that can achieve contact and separation between the conductive ring 841 and the fixed wiring portion 842 is within the scope of protection of the present disclosure.

In another embodiment, the fixed wiring portion 842 includes a bracket 8423 and a circuit board 8424 fixed to the bracket 8423.

The bracket 8423 is rotatably mounted with the conductive ring 841 through a bearing 843, and an opening 84231 is formed in a sidewall of the bracket 8423.

The circuit board 8424 is mounted in the opening 84231, and electrical connecting ends 84241 are formed on one side of the circuit board 8424 towards the conductive ring 841, and are always in contact with a sidewall of the conductive ring 841 to achieve electric conduction in a process that the conductive ring 841 rotates relative to the bracket 8423.

In an embodiment, the hypochlorous acid sterilization apparatus 830 is mounted on a sidewall of the inner drum 811. Specifically, the hypochlorous acid sterilization apparatus 830 may be selectively mounted on the inner sidewall of the inner drum 811 and the outer sidewall of the inner drum 811. Water flows through the hypochlorous acid sterilization apparatus 830 and then is electrolyzed by the hypochlorous acid sterilization apparatus to generate slightly acidic electrolyzed water, thus further improving the cleaning ability of the washing water. In addition, the slightly acidic electrolyzed water can not only sterilize the clothes inside the drum, but also sterilize the drum body 810 of the washing machine, and cooperate with the rotating inner drum 811 to expand the coverage of the sterilization and purification effect. For example, when the hypochlorous acid sterilization apparatus 830 is mounted on the inner sidewall of the inner drum 811, the generated slightly acidic electrolyzed water directly contacts the clothes, thus further improving the sterilization effect on the clothes. When the hypochlorous acid sterilization apparatus 830 is mounted on the outer sidewall of the inner drum 811, the slightly acidic electrolyzed water generated in a space sandwiched between the inner drum 811 and the outer tub 812 enters the inner drum 811 through a water hole in the inner drum 811. In this way, the washing environment inside the drum body 810 may be sterilized and cleaned while the clothes is sterilized, thus maintaining the cleanliness of the washing environment.

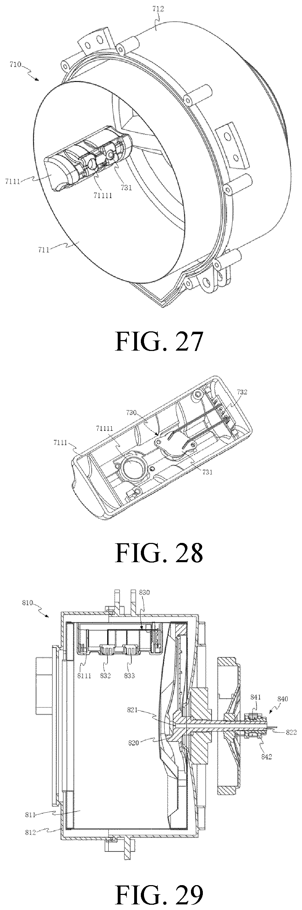

In an embodiment, as shown in FIG. 29 to FIG. 31, the inner drum 811 further includes a lifter 8111, and the hypochlorous acid sterilization apparatus 830 is mounted in the lifter 8111. Specifically, the washing water enters the lifter 8111, is electrolyzed by the hypochlorous acid sterilization apparatus 830, and flows back into the drum to clean the clothes. On the one hand, the arrangement that the hypochlorous acid sterilization apparatus 830 is hidden inside the lifter 8111 optimizes the structural layout of the inner drum 811, and is conducive to the attractiveness of the inner drum 811. On the other hand, the sterilization and purification effect of the hypochlorous acid sterilization apparatus 830 cooperates with the smashing and stirring effect of the lifter 8111 on the clothes, thus optimizing the washing effect.

Further, the hypochlorous acid sterilization apparatus 830 includes an electrolysis circuit board 831, a positive electrode end 832 and a negative electrode end 833.

The electrolysis circuit board 831 is clamped on an inner side of the lifter 8111 and is connected with a wiring end of the power supply apparatus 840. The positive electrode end 832 and the negative electrode end 833 extend from a surface of the lifter 8111 and are respectively connected with a positive electrode and a negative electrode of the electrolysis circuit board 831.

Further, the surface of the lifter 8111 is provided with two through holes 81111 for respectively clamping the positive electrode end 832 and the negative electrode end 833. Specifically, the shapes of the through holes 81111 adapt to the shapes of the positive electrode end 832 and the negative electrode end 833, making it easy to mount while maintaining good stability.

In an embodiment, the hypochlorous acid sterilization apparatus 830 is mounted at the bottom of the inner drum 811. Specifically, the hypochlorous acid sterilization apparatus 830 may be mounted on an inner side or outer side of the bottom of the drum. Preferably, the hypochlorous acid sterilization apparatus 830 may be mounted at the bottom of the drum near the sidewall, thus ensuring sufficient contact between the washing water and the hypochlorous acid sterilization apparatus 830 during the rotation or non-rotation of the inner drum 811. Further, the hypochlorous acid sterilization apparatus 830 may also be mounted on the tripod 820.

A washing machine includes the internal drum body 810 of the washing machine described above.

In this embodiment, the power supply apparatus 840 can supply power to the hypochlorous acid sterilization apparatus 830 mounted on the inner drum 811 when the inner drum 811 rotates, water is electrolyzed by the hypochlorous acid sterilization apparatus 830, then slightly acidic electrolyzed water is generated. The slightly acidic electrolyzed water has strong oxidation ability and fast and efficient microbial killing effect, and can sterilize the environment inside the drum, including the clothes inside the drum when the inner drum 811 rotates, so as to improve the cleanliness of the drum body 810 and the clothes, and achieve high safety. In this way, the cooperation between the rotating inner drum 811 and the hypochlorous acid sterilization apparatus 830 may further expand functions of the washing machine, optimize overall functions of the washing machine, meet different needs of users, and improve user experience. In addition, the arrangement that the wiring end of the power supply apparatus 840 penetrates through the hollow portion inside the tripod 820 can effectively avoid entanglement of conducting wires during rotation, the structure is simple, the operation is stable, and the reliability is high. The power supply apparatus 840 is the apparatus for supplying power to the interior of the drum body of the drum washing machine in Embodiment 1 or Embodiment 3.

### Embodiment 9

This embodiment provides an internal drum body 910 of a washing machine. As shown in FIG. 16 to FIG. 18 and FIG. 32 to FIG. 33, the internal drum body includes:
an inner drum 911, configured to accommodate clothes;
a tripod 920, fixedly connected with a bottom of the inner drum 911 to transmit a rotation driving force to the inner drum 911;
a steam generation apparatus 930, disposed in the inner drum 911 to provide steam to the drum body 910; and
a power supply apparatus 940, located at one end of the tripod 920, where a wiring end of the power supply apparatus 940 penetrates through a hollow portion inside the tripod 920 and is connected with the steam generation apparatus 930.

The power supply apparatus 940 is configured to maintain power supply to the steam generation apparatus 930 in a process that the tripod 920 drives the inner drum 911 to rotate.

**In** this embodiment, the power supply apparatus 940 can supply power to the steam generation apparatus 930 mounted on the inner drum 911 when the inner drum 911 rotates, and a steam generator is utilized to generate steam for clothes care or high-temperature sterilization. **In** this way, the cooperation between the rotating inner drum 911 and the steam generation apparatus 930 may further expand functions of the washing machine, optimize overall functions of the washing machine, meet different needs of users, and improve user experience. **In** addition, the arrangement that the wiring end of the power supply apparatus 940 penetrates through the hollow portion inside the tripod 920 can effectively avoid entanglement of conducting wires during rotation, the structure is simple, the operation is stable, and the reliability is high.

The power supply apparatus 940 is the apparatus for supplying power to the interior of the drum body of the drum washing machine in Embodiment 1 or Embodiment 3.

Further, the power supply apparatus 940 includes a conductive ring 941 and a fixed wiring portion 942 which are electrically connected.

The conductive ring 941 is fixed at an end portion of a rotating shaft 922 of the tripod 920 and synchronously rotates along with the tripod 920.

The fixed wiring portion 942 is mounted with a housing located outside the inner drum 911 and is configured to contact the conductive ring 941 to achieve electrical connection, so as to achieve electric conduction in a process that the conductive ring 941 rotates relative to the fixed wiring portion 942.

Further, the conductive ring 941 includes a plurality of metal conductive areas 9411 and a plurality of conducting wires 9412 corresponding to the metal conductive areas 9411.

An insulating plate 9413 is provided between adjacent metal conductive areas 9411.

One end of each conducting wire 9412 is electrically connected with the corresponding metal conductive area 9411, and the other end penetrates through a wiring passage 921 inside the tripod 920 to enter the inner drum 911 and connect with a power supply end of the steam generation apparatus 930.

In an embodiment, the fixed wiring portion 942 includes a telescopic electromagnet 9421 and a pogopin circuit board 9422. The telescopic electromagnet 9421 includes an electromagnet body 94211, a cylindrical protrusion, a spring, and a telescopic rod 94212. A center of the electromagnet body 94211 is provided with a mounting hole. One end of the telescopic rod 94212 penetrates through the mounting hole and is provided with the spring at the end portion, and the cylindrical protrusion is mounted at the other end of the telescopic rod. The cylindrical protrusion serves as an output end of the telescopic electromagnet 9421. The pogopin circuit board 9422 is mounted to the cylindrical protrusion.

The working flow of the telescopic electromagnet 9421 is as follows:
Power off: when it is not powered on, the telescopic electromagnet 9421 is in a non-working state, and the telescopic rod 94212 is in a non-popping-out state and is retracted in the electromagnet.
Power on: when a main control board of the washing machine is powered on, the telescopic electromagnet 9421 is in a working state, the telescopic rod 94212 is subjected to the magnetic force of the electromagnet, the magnetic force is greater than the elastic force of the spring, the telescopic rod 94212 pops out and maintains the set stroke distance.

It is to be noted that the fixed wiring portion 942 is of a telescopic structure. For different inner drum structures and actual design requirements of washing machines, the fixed wiring portion 942 may also be arranged as a swinging structure, a rotating structure, or a linear moving structure driven by a motor, an electric push rod, or any other driving apparatus. That is, the solution that can achieve contact and separation between the conductive ring 941 and the fixed wiring portion 942 is within the scope of protection of the present disclosure.

In another embodiment, the fixed wiring portion 942 includes a bracket 9423 and a circuit board 9424 fixed to the bracket 9423.

The bracket 9423 is rotatably mounted with the conductive ring 941 through a bearing 943, and an opening 94231 is formed in a sidewall of the bracket 9423.

The circuit board 9424 is mounted in the opening 94231, and electrical connecting ends 94241 are formed on one side of the circuit board 9424 towards the conductive ring 941, and are always in contact with a sidewall of the conductive ring 941 to achieve electric conduction in a process that the conductive ring 941 rotates relative to the bracket 9423.

In an embodiment, the steam generation apparatus 930 is a thick film steam generator. The thick film steam generator used has a small volume, high heat density, and high efficiency in generating steam, and can continuously generate steam. It is to be noted that the steam generation apparatus 930 may also be configured as an electric heating tube or electric heating disc steam generator to directly provide high-temperature steam to the environment in the inner drum, which can participate in the washing process or high-temperature sterilization process.

In an embodiment, the steam generation apparatus 930 is mounted on a sidewall of the inner drum 911. Specifically, the steam generation apparatus 930 may be selectively mounted on the inner wall of the inner drum 911 or the outer wall of the inner drum 911, the washing water is heated by the steam generation apparatus 930 to form steam, and the steam acts on the clothes inside the drum body 910 during the rotation or non-rotation of the inner drum 911. For example, when the steam generation apparatus 930 is mounted on the inner wall of the inner drum 911, steam is directly formed inside the inner drum 911 and acts directly on the clothes; and when the steam generation apparatus 930 is mounted on the outer wall of the inner drum 911, steam is formed in a space between the inner drum 911 and the outer tub 912.

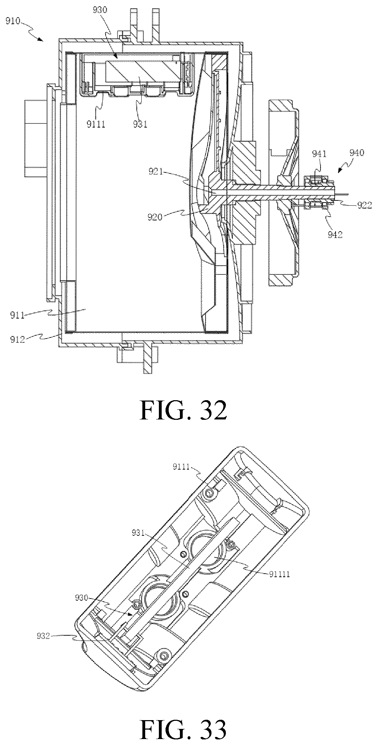

In an embodiment, as shown in FIG. 32 and FIG. 33, the inner drum 911 further includes a lifter 9111, and the steam generation apparatus 930 is mounted in the lifter 9111. Specifically, the lifter 9111 is provided with a through hole 91111. The washing water enters the lifter 9111 through the through hole 91111 and is heated by the steam generation apparatus 930 to form steam, and then the steam is discharged through the through hole 91111. On the one hand, the arrangement that the steam generation apparatus 930 is hidden inside the lifter 9111 optimizes the structural layout of the inner drum 911, and the lifter 9111 forms a protective outer shell of the steam generation apparatus 930 and is conducive to the attractiveness of the inner drum 911. On the other hand, the heating effect of the steam generation apparatus 930 cooperates with the smashing and stirring effect of the lifter 9111 on the clothes, thus optimizing the washing effect.

Further, the steam generation apparatus 930 includes a thick film heating plate 931, and the thick film heating plate extends along a length direction of the lifter 9111.

Specifically, the steam generation apparatus 930 includes a thick film heating plate 931 and a heating circuit board 932 connected with the thick film heating plate 931. The heating circuit board 932 is mounted inside the lifter 9111 and connected with the main control board of the washing machine. The thick film heating plate 931 extends along a length direction of the lifter 9111. This arrangement is conducive to increasing a heating area and improving a contact area between the washing water and the heating tube, thus improving the heating efficiency and optimizing the washing effect.

In an embodiment, the steam generation apparatus 930 is mounted at the bottom of the inner drum 911. The steam generation apparatus 930 may be mounted on the inner wall of the bottom of the drum or the outer wall of the bottom of the drum, the washing water is heated by the steam generation apparatus 930 to form steam, and the steam acts on the clothes inside the drum body 910 during the rotation or non-rotation of the inner drum 911, thus enhancing the washing effect. Further, the steam generation apparatus 930 may also be mounted on the tripod 920 and face towards the inner drum 911.

A washing machine includes the internal drum body 910 of the washing machine described above. In this embodiment, the power supply apparatus 940 can supply power to the steam generation apparatus 930 mounted on the inner drum 911 when the inner drum 911 rotates, and a steam generator is utilized to generate steam for clothes care. In this way, the cooperation between the rotating inner drum 911 and the steam generation apparatus 930 may further expand functions of the washing machine, optimize overall functions of the washing machine, meet different needs of users, and improve user experience. In addition, the arrangement that the wiring end of the power supply apparatus 940 penetrates through the hollow portion inside the tripod 920 can effectively avoid entanglement of conducting wires during rotation, the structure is simple, the operation is stable, and the reliability is high.

What are described above are just exemplary implementations of the present disclosure. It is to be pointed out that those of ordinary skill in the art may make several improvements and modifications without departing from the principle of the present disclosure, which, however, still fall within the scope of protection of the present disclosure.

## Claims

1. An apparatus for supplying power to interior of a drum body of a drum washing machine, the drum washing machine comprising an inner drum (14), an outer tub (13), and a main control board of the washing machine, wherein the apparatus comprises a telescopic electromagnet (1), a pogopin circuit board (2), a locking nut (3), a conductive ring (4), a fixing block (5), a tripod (6), and a supporting frame (8), wherein the supporting frame (8) is provided on an outer wall of the outer tub (13), an inner drum rib protruding to the interior of the inner drum is provided on an inner wall of the inner drum (14), a plurality of functional assemblies are provided in the inner drum (14), a bottom end of the tripod (6) is fixed on an outer side of the inner drum (14), and a top end penetrates through the outer tub (13) and is fixedly connected with the supporting frame (8);
the fixing block (5) penetrates through the supporting frame (8) and is connected with the top end of the tripod (6), an outer side of the fixing block (5) is provided with the locking nut (3), the conductive ring (4) is mounted on the fixing block (5), the telescopic electromagnet (1) is mounted on the supporting frame (8), the telescopic electromagnet (1) and the conductive ring (4) are located at opposite positions, the pogopin circuit board (2) is mounted at an output end of the telescopic electromagnet (1), the telescopic electromagnet (1) and the pogopin circuit board (2) are both electrically connected with the main control board of the washing machine, and the telescopic electromagnet (1) extends or retracts to drive the pogopin circuit board (2) to change a position to connect or separate the pogopin circuit board (2) and the conductive ring (4).

2. The apparatus for supplying power to the interior of the drum body of the drum washing machine according to claim 1, wherein the pogopin circuit board (2) is provided with a plurality of pogopins, the conductive ring (4) is provided with a plurality of metal conductive sheets (12) corresponding to the pogopins and a plurality of conductive electric wires (11) corresponding to the metal conductive sheets (12), insulating plastic (10) is provided between adjacent metal conductive sheets (12), one ends of the conductive electric wires (11) are electrically connected with the metal conductive sheets (12), and the other ends penetrate through the supporting frame (8) and a central passage in the tripod (6), enter an outer side of the inner drum rib and are respectively connected with power supply ends of the functional assemblies.

3. The apparatus for supplying power to the interior of the drum body of the drum washing machine according to claim 2, wherein the metal conductive sheets (12) are of a ring-shaped structure, and the conductive electric wires (11) penetrate through a central hole of the metal conductive sheets (12) and enter the central passage of the tripod (6), then the central passage is sealed with liquid silica gel, and the silica gel is solidified to seal the central passage.

4. The apparatus for supplying power to the interior of the drum body of the drum washing machine according to claim 2, wherein the pogopin circuit board (2) is provided with three pogopins, and the conductive ring (4) is provided with three metal conductive sheets (12) and three conductive electric wires (11).

5. The apparatus for supplying power to the interior of the drum body of the drum washing machine according to claim 1, wherein the apparatus further comprises a DD motor (9) configured to drive the inner drum (14) to rotate, a motor rotor (7) of the DD motor (9) sleeves the tripod (6), the DD motor (9) is mounted between the outer tub (13) and the supporting frame (8), and a rotation speed of the DD motor (9) is fed back to the main control board of the washing machine in real time; and
the main control board of the washing machine controls the on/off of the power supply to the telescopic electromagnet (1) and the pogopin circuit board according to a current washing process and a state of the DD motor (9).

6. The apparatus for supplying power to the interior of the drum body of the drum washing machine according to claim 1, wherein the telescopic electromagnet (1) comprises an electromagnet body (101), a cylindrical protrusion (102), a spring (103), and a telescopic rod (104), a center of the electromagnet body (101) is provided with a through hole, one end of the telescopic rod (104) penetrates through the through hole and is provided with the spring (103) at an end portion, the cylindrical protrusion (102) is mounted at the other end, and the cylindrical protrusion (102) serves as the output end of the telescopic electromagnet (1).

7. An apparatus for supplying power to interior of a drum body of a drum washing machine, comprising:
an inner drum, configured to accommodate clothes;
an outer tub, mounted at an outer side of the inner drum;
a tripod, fixedly connected with a bottom of the inner drum to transmit a rotation driving force to the inner drum, wherein an end portion of the tripod extends along an axial direction of the inner drum and penetrates through the outer tub to form a rotating shaft;
a conductive ring, fixed to an end portion of the rotating shaft and electrically conducted with a functional assembly inside the drum body of the washing machine;
a bearing, wherein an inner ring is fitted and fixed to an outer wall of the conductive ring, and the bearing is configured to achieve synchronous rotation of the conductive ring with the inner ring of the bearing; and
at least one circuit board assembly, electrically connected with a main control board of the washing machine and mounted on an outer ring of the bearing, wherein electrical connecting ends are formed on the circuit board assembly and configured to be connected with the conductive ring,
wherein in a process that the conductive ring rotates relative to the circuit board assembly, the electrical connecting ends are configured to be always in contact with the conductive ring to achieve power supply to the functional assembly inside the drum body of the washing machine.

8. The apparatus for supplying power to the interior of the drum body of the drum washing machine according to claim 7, wherein a surface of the conductive ring in contact with the electrical connecting ends is provided with a coating layer.

9. The apparatus for supplying power to the interior of the drum body of the drum washing machine according to claim 7, wherein a plurality of metal conductive areas with positions corresponding to the electrical connecting ends and a plurality of conducting wires corresponding to the metal conductive areas are formed on the conductive ring, wherein
an insulating plate is provided between adjacent metal conductive areas;
one end of each conducting wire is electrically connected with the corresponding metal conductive area, and the other end penetrates through a wiring passage inside the tripod to enter the outer side of the inner drum and connect with a power supply end of the functional assembly.

10. The apparatus for supplying power to the interior of the drum body of the drum washing machine according to claim 7, wherein the tripod comprises a tripod body and the rotating shaft, and the tripod body is fixedly connected with the bottom of the inner drum, wherein
a first wiring space is formed inside the rotating shaft, and a second wiring space is formed inside the tripod body; and the conducting wires sequentially penetrate through the first wiring space and the second wiring space, and exit from an end portion of the tripod body.

11. The apparatus for supplying power to the interior of the drum body of the drum washing machine according to claim 7, wherein the circuit board assembly comprises a bracket and a circuit board mounted on the bracket, wherein
the bracket is fixed to the outer ring of the bearing, and an opening is formed in a sidewall of the bracket; and
the electrical connecting ends are formed on the circuit board, and the circuit board is mounted in the opening to make the electrical connecting ends be in contact with the conductive ring.

12. The apparatus for supplying power to the interior of the drum body of the drum washing machine according to claim 7, wherein the electrical connecting ends are elastic members, and end portions of the elastic members are maintained in contact with the conductive ring under an elastic action.

13. The apparatus for supplying power to the interior of the drum body of the drum washing machine according to claim 7, further comprising: an end cover, mounted at an end portion of the conductive ring and configured to fix the inner ring of the bearing from an outer side.

14. An internal drum body of a washing machine, comprising:
an inner drum, configured to accommodate clothes;
a tripod, fixedly connected with a bottom of the inner drum to transmit a rotation driving force to the inner drum;
a power actuating mechanism, disposed inside the inner drum to actuate a traction effect on the clothes; and
a power supply apparatus, located at one end of the tripod, wherein a wiring end of the power supply apparatus penetrates through a hollow portion inside the tripod and is connected with the power actuating mechanism,
wherein the power supply apparatus is configured to maintain power supply to the power actuating mechanism in a process that the tripod drives the inner drum to rotate.

15. The internal drum body of the washing machine according to claim 14, wherein the power actuating mechanism comprises one or more of a gripping apparatus, a poking apparatus, a stirring apparatus, and a lifting apparatus.

16. The internal drum body of the washing machine according to claim 14, wherein the power supply apparatus comprises a conductive ring and a fixed wiring portion which are electrically connected, wherein
the conductive ring is fixed at an end portion of the tripod and synchronously rotates along with the tripod; and
the fixed wiring portion is mounted with a housing located outside the inner drum and is configured to contact the conductive ring to achieve electrical connection, so as to achieve electric conduction in a process that the conductive ring rotates relative to the fixed wiring portion.

17. The internal drum body of the washing machine according to claim 16, wherein the fixed wiring portion comprises a telescopic electromagnet and a pogopin circuit board, wherein
the pogopin circuit board is mounted at an output end of the telescopic electromagnet; and
the telescopic electromagnet extends and retracts to drive the pogopin circuit board to produce a positional change, so that the pogopin circuit board is connected with or separated from the conductive ring.

18. The internal drum body of the washing machine according to claim 16, wherein the fixed wiring portion comprises a bracket and a circuit board fixed to the bracket, wherein
the bracket is rotatably mounted with the conductive ring through a bearing; and
electrical connecting ends are formed on one side of the circuit board towards the conductive ring, and are always in contact with a sidewall of the conductive ring to achieve electric conduction in a process that the conductive ring rotates relative to the bracket.

19. The internal drum body of the washing machine according to claim 17 or 18, wherein the power actuating mechanism is mounted on a sidewall of the inner drum.

20. The internal drum body of the washing machine according to claim 17 or 18, wherein the inner drum further comprises a lifter, and the power actuating mechanism is mounted with the lifter.

21. The internal drum body of the washing machine according to claim 20, wherein the power actuating mechanism is hidden in the lifter.

22. The internal drum body of the washing machine according to claim 17 or 18, wherein the power actuating mechanism is mounted at the bottom of the inner drum.

23. An internal drum body of a washing machine, comprising:
an inner drum, configured to accommodate clothes;
a tripod, fixedly connected with a bottom of the inner drum to transmit a rotation driving force to the inner drum;
a sensing apparatus, disposed in the inner drum to sense environmental parameters in the inner drum; and
a power supply apparatus, located at one end of the tripod, wherein a wiring end of the power supply apparatus penetrates through a hollow portion inside the tripod and is connected with the sensing apparatus,
wherein the power supply apparatus is configured to maintain power supply to the sensing apparatus in a process that the tripod drives the inner drum to rotate.

24. The internal drum body of the washing machine according to claim 23, wherein the sensing apparatus comprises one or more of a temperature sensor, a humidity sensor, a weight sensor, an acceleration sensor, a position sensor, a water level sensor, and a water quality sensor.

25. The internal drum body of the washing machine according to claim 23, wherein the power supply apparatus comprises a conductive ring and a fixed wiring portion which are electrically connected, wherein
the conductive ring is fixed at an end portion of the tripod and synchronously rotates along with the tripod; and
the fixed wiring portion is mounted with a housing located outside the inner drum and is configured to contact the conductive ring to achieve electrical connection, so as to achieve electric conduction in a process that the conductive ring rotates relative to the fixed wiring portion.

26. The internal drum body of the washing machine according to claim 25, wherein the fixed wiring portion comprises a telescopic electromagnet and a pogopin circuit board, wherein
the pogopin circuit board is mounted at an output end of the telescopic electromagnet; and
the telescopic electromagnet extends and retracts to drive the pogopin circuit board to produce a positional change, so that the pogopin circuit board is connected with or separated from the conductive ring.

27. The internal drum body of the washing machine according to claim 25, wherein the fixed wiring portion comprises a bracket and a circuit board fixed to the bracket, wherein
the bracket is rotatably mounted with the conductive ring through a bearing; and
electrical connecting ends are formed on one side of the circuit board towards the conductive ring, and are always in contact with a sidewall of the conductive ring to achieve electric conduction in a process that the conductive ring rotates relative to the bracket.

28. The internal drum body of the washing machine according to claim 26 or 27, wherein the sensing apparatus is mounted on a sidewall of the inner drum.

29. The internal drum body of the washing machine according to claim 26 or 27, wherein the inner drum further comprises a lifter, and the sensing apparatus is fixedly mounted with the lifter.

30. The internal drum body of the washing machine according to claim 29, wherein the sensing apparatus comprises at least one sensing area, and the sensing area is embedded into a surface of the lifter.

31. The internal drum body of the washing machine according to claim 26 or 27, wherein the sensing apparatus is mounted at the bottom of the inner drum.

32. An internal drum body of a washing machine, comprising:
an inner drum, configured to accommodate clothes;
a tripod, fixedly connected with a bottom of the inner drum to transmit a rotation driving force to the inner drum;
a heating apparatus, disposed in the inner drum to provide a thermal environment of the drum body; and
a power supply apparatus, located at one end of the tripod, wherein a wiring end of the power supply apparatus penetrates through a hollow portion inside the tripod and is connected with the heating apparatus,
wherein the power supply apparatus is configured to maintain power supply to the heating apparatus in a process that the tripod drives the inner drum to rotate.

33. The internal drum body of the washing machine according to claim 32, wherein the power supply apparatus comprises a conductive ring and a fixed wiring portion which are electrically connected, wherein
the conductive ring is fixed at an end portion of the tripod and synchronously rotates along with the tripod; and
the fixed wiring portion is mounted with a housing located outside the inner drum and is configured to contact the conductive ring to achieve electrical connection, so as to achieve electric conduction in a process that the conductive ring rotates relative to the fixed wiring portion.

34. The internal drum body of the washing machine according to claim 33, wherein the fixed wiring portion comprises a telescopic electromagnet and a pogopin circuit board, wherein
the pogopin circuit board is mounted at an output end of the telescopic electromagnet; and
the telescopic electromagnet extends and retracts to drive the pogopin circuit board to produce a positional change, so that the pogopin circuit board is connected with or separated from the conductive ring.

35. The internal drum body of the washing machine according to claim 33, wherein the fixed wiring portion comprises a bracket and a circuit board fixed to the bracket, wherein
the bracket is rotatably mounted with the conductive ring through a bearing; and
electrical connecting ends are formed on one side of the circuit board towards the conductive ring, and are always in contact with a sidewall of the conductive ring to achieve electric conduction in a process that the conductive ring rotates relative to the bracket.

36. The internal drum body of the washing machine according to claim 34 or 35, wherein the heating apparatus is mounted on a sidewall of the inner drum.

37. The internal drum body of the washing machine according to claim 34 or 35, wherein the inner drum further comprises a lifter, and the heating apparatus is mounted in the lifter.

38. The internal drum body of the washing machine according to claim 37, wherein the heating apparatus comprises a heating tube, and the heating tube extends along a length direction of the lifter.

39. The internal drum body of the washing machine according to claim 34 or 35, wherein the heating apparatus is mounted at the bottom of the inner drum.

40. The internal drum body of the washing machine according to claim 34 or 35, wherein the heating apparatus is arranged close to the tripod.

41. An internal drum body of a washing machine, comprising:
an inner drum, configured to accommodate clothes;
a tripod, fixedly connected with a bottom of the inner drum to transmit a rotation driving force to the inner drum;
a sterilization apparatus, disposed inside the inner drum to perform sterilization and purification on the drum body; and
a power supply apparatus, located at one end of the tripod, wherein a wiring end of the power supply apparatus penetrates through a hollow portion inside the tripod and is connected with the sterilization apparatus,
wherein the power supply apparatus is configured to maintain power supply to the sterilization apparatus in a process that the tripod drives the inner drum to rotate.

42. The internal drum body of the washing machine according to claim 41, wherein the power supply apparatus comprises a conductive ring and a fixed wiring portion which are electrically connected, wherein
the conductive ring is fixed at an end portion of the tripod and synchronously rotates along with the tripod; and
the fixed wiring portion is mounted with a housing located outside the inner drum and is configured to contact the conductive ring to achieve electrical connection, so as to achieve electric conduction in a process that the conductive ring rotates relative to the fixed wiring portion.

43. The internal drum body of the washing machine according to claim 42, wherein the fixed wiring portion comprises a telescopic electromagnet and a pogopin circuit board, wherein
the pogopin circuit board is mounted at an output end of the telescopic electromagnet; and
the telescopic electromagnet extends and retracts to drive the pogopin circuit board to produce a positional change, so that the pogopin circuit board is connected with or separated from the conductive ring.

44. The internal drum body of the washing machine according to claim 42, wherein the fixed wiring portion comprises a bracket and a circuit board fixed to the bracket, wherein
the bracket is rotatably mounted with the conductive ring through a bearing; and
electrical connecting ends are formed on one side of the circuit board towards the conductive ring, and are always in contact with a sidewall of the conductive ring to achieve electric conduction in a process that the conductive ring rotates relative to the bracket.

45. The internal drum body of the washing machine according to any one of claims 41 to 44, wherein the sterilization apparatus is a light sterilization apparatus.

46. The internal drum body of the washing machine according to claim 45, wherein the light sterilization apparatus is mounted on an inner sidewall of the inner drum to sterilize the clothes and washing water in the inner drum.

47. The internal drum body of the washing machine according to claim 45, wherein the light sterilization apparatus is mounted on an outer sidewall of the inner drum to sterilize a space sandwiched between the inner drum and the outer tub.

48. The internal drum body of the washing machine according to claim 45, wherein the inner drum further comprises a lifter, and the light sterilization apparatus is mounted in the lifter.

49. The internal drum body of the washing machine according to claim 45, wherein the light sterilization apparatus is mounted at the bottom of the inner drum.

50. The internal drum body of the washing machine according to any one of claims 46 to 49, wherein the light sterilization apparatus comprises a UV sterilization apparatus or a light plasma sterilization apparatus.

51. The internal drum body of the washing machine according to any one of claims 41 to 44, wherein the sterilization apparatus is a hypochlorous acid sterilization apparatus.

52. The internal drum body of the washing machine according to claim 51, wherein the hypochlorous acid sterilization apparatus is mounted on a sidewall of the inner drum.

53. The internal drum body of the washing machine according to claim 51, wherein the inner drum further comprises a lifter, and the hypochlorous acid sterilization apparatus is mounted in the lifter.

54. The internal drum body of the washing machine according to claim 53, wherein the hypochlorous acid sterilization apparatus comprises an electrolysis circuit board, a positive electrode end, and a negative electrode end, wherein
the electrolysis circuit board is clamped on an inner side of the lifter and is connected with a wiring end of the power supply apparatus; the positive electrode end and the negative electrode end extend from a surface of the lifter and are respectively connected with a positive electrode and a negative electrode of the circuit board.

55. The internal drum body of the washing machine according to claim 54, wherein the surface of the lifter is provided with two through holes for respectively clamping the positive electrode end and the negative electrode end.

56. The internal drum body of the washing machine according to claim 51, wherein the hypochlorous acid sterilization apparatus is mounted at the bottom of the inner drum.

57. The internal drum body of the washing machine according to any one of claims 41 to 44, wherein the sterilization apparatus is a high-temperature steam sterilization apparatus.

58. The internal drum body of the washing machine according to claim 57, wherein the high-temperature steam sterilization apparatus is mounted on a sidewall of the inner drum.

59. The internal drum body of the washing machine according to claim 57, wherein the inner drum further comprises a lifter, and the high-temperature steam sterilization apparatus is mounted in the lifter.

60. The internal drum body of the washing machine according to claim 59, wherein the high-temperature steam sterilization apparatus comprises a heating plate, and the heating plate extends along a length direction of the lifter.

61. The internal drum body of the washing machine according to claim 55, wherein the high-temperature steam sterilization apparatus is mounted at the bottom of the inner drum.

62. An internal drum body of a washing machine, comprising:
an inner drum, configured to accommodate clothes;
a tripod, fixedly connected with a bottom of the inner drum to transmit a rotation driving force to the inner drum;
a steam generation apparatus, disposed in the inner drum to provide steam to the drum body; and
a power supply apparatus, located at one end of the tripod, wherein a wiring end of the power supply apparatus penetrates through a hollow portion inside the tripod and is connected with the steam generation apparatus,
wherein the power supply apparatus is configured to maintain power supply to the steam generation apparatus in a process that the tripod drives the inner drum to rotate.

63. The internal drum body of the washing machine according to claim 62, wherein the power supply apparatus comprises a conductive ring and a fixed wiring portion which are electrically connected, wherein
the conductive ring is fixed at an end portion of the tripod and synchronously rotates along with the tripod; and
the fixed wiring portion is mounted with a housing located outside the inner drum and is configured to contact the conductive ring to achieve electrical connection, so as to achieve electric conduction in a process that the conductive ring rotates relative to the fixed wiring portion.

64. The internal drum body of the washing machine according to claim 63, wherein the fixed wiring portion comprises a telescopic electromagnet and a pogopin circuit board, wherein
the pogopin circuit board is mounted at an output end of the telescopic electromagnet; and
the telescopic electromagnet extends and retracts to drive the pogopin circuit board to produce a positional change, so that the pogopin circuit board is connected with or separated from the conductive ring.

65. The internal drum body of the washing machine according to claim 63, wherein the fixed wiring portion comprises a bracket and a circuit board fixed to the bracket, wherein
the bracket is rotatably mounted with the conductive ring through a bearing; and
electrical connecting ends are formed on one side of the circuit board towards the conductive ring, and are always in contact with a sidewall of the conductive ring to achieve electric conduction in a process that the conductive ring rotates relative to the bracket.

66. The internal drum body of the washing machine according to claim 64 or 65, wherein the steam generation apparatus is mounted on a sidewall of the inner drum.

67. The internal drum body of the washing machine according to claim 64 or 65, wherein the inner drum further comprises a lifter, and the steam generation apparatus is mounted in the lifter.

68. The internal drum body of the washing machine according to claim 67, wherein the steam generation apparatus comprises a heating plate, and the heating plate extends along a length direction of the lifter.

69. The internal drum body of the washing machine according to claim 64 or 65, wherein the steam generation apparatus is mounted at the bottom of the inner drum.

70. A washing machine, comprising the apparatus for supplying power to the interior of the drum body of the drum washing machine according to any one of claims 1 to 6 or any one of claims 7 to 13; or comprising the internal drum body of the washing machine according to any one of claims 14 to 22, or any one of claims 23 to 31, or any one of claims 32 to 40, or any one of claims 41 to 61, or any one of claims 62 to 69.
